(19) Europäisches Patentamt

European Patent Office

Office européen des brevets

(11) **EP 1 941 062 B1**

(12) **FASCICULE DE BREVET EUROPEEN**

(45) Date de publication et mention
de la délivrance du brevet:
**16.12.2009 Bulletin 2009/51**

(21) Numéro de dépôt: **06831008.5**

(22) Date de dépôt: **25.10.2006**

(51) Int Cl.:
*C13F 3/00* *(2006.01)*      *A61K 9/20* *(2006.01)*
*C13F 5/00* *(2006.01)*

(86) Numéro de dépôt international:
**PCT/FR2006/002393**

(87) Numéro de publication internationale:
**WO 2007/048922 (03.05.2007 Gazette 2007/18)**

(54) **PROCEDE DE PREPARATION D'UNE COMPOSITION DE SACCHAROSE, PRODUIT TEL QU' OBTENU ET SON UTILISATION**

VERFAHREN ZUR HERSTELLUNG EINER SACCHAROSE-ZUSAMMENSETZUNG, DARAUS RESULTIERENDES PRODUKT UND ANWENDUNG DAVON

METHOD FOR PRODUCING A SACCHAROSE COMPOSITION, RESULTING PRODUCT AND USE THEREOF

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **28.10.2005 FR 0511070**

(43) Date de publication de la demande:
**09.07.2008 Bulletin 2008/28**

(73) Titulaire: **TEREOS**
**02390 Origny Sainte Benoite (FR)**

(72) Inventeurs:
• **WONG, Emile**
**F-01700 Neyron (FR)**

• **DELHORBE, Philippe**
**F-59139 Wattignies (FR)**

(74) Mandataire: **Grosset-Fournier, Chantal Catherine et al**
**Grosset-Fournier & Demachy**
**54, Rue Saint-Lazare**
**75009 Paris (FR)**

(56) Documents cités:
**EP-A- 0 334 617**     **WO-A-02/06538**
**GB-A- 1 386 378**     **US-A- 5 593 502**
**US-A- 5 980 941**     **US-A1- 2004 208 981**

## Description

La présente invention a pour objet un procédé de préparation d'une composition de saccharose, ainsi que le produit obtenu selon ledit procédé. L'invention concerne également l'utilisation dudit produit, par exemple, pour la préparation de comprimés, notamment de comprimés pharmaceutiques.

Les poudres à compression directe à base de saccharose actuellement sur le marché contiennent au moins un liant ou additif qui confère au sucre ses propriétés de compression.

Plusieurs produits sont actuellement utilisés en tant que sucre directement compressible et sont obtenus par co-cristallisation (Domino Dipac®), par agglomération en lit fluidisé (Südzucker Compri-Sugar®), par granulation humide (Tereos Alvéo-sucre®) ou par compactage (Chr. Hansen Nu-Tab®).

Il n'existe pas à ce jour de poudre de saccharose ayant des propriétés de compression directe, dans la mesure où notamment tous les procédés de préparation à ce jour de sucre à compression directe sont inapplicables à l'utilisation de saccharose seul.

Les procédés de fabrication des sucres à compression directe peuvent être classés en quatre catégories : agglomération, atomisation, compactage et microcristallisation.

Un procédé d'agglomération (granulation humide et séchage) correspond à un procédé dans lequel le sucre sous forme solide (souvent du sucre glace) est aggloméré par une solution de liant, souvent ajoutée par pulvérisation suivie d'un séchage par air chaud.

La technique est en fait un procédé d'agglomération de particules de petites dimensions avec le liant, qui entraîne une augmentation de la taille des particules par agglomération.

C'est le procédé utilisé notamment pour l'Alvéosucre® (Tereos) ou pour le Compri-Sugar® (Südzucker).

A ce titre, le brevet européen 0 334 617 décrit un procédé d'obtention d'un nouvel édulcorant, sous la forme de sphères creuses, comprenant l'atomisation d'une solution de sucre et en présence de $CO_2$ comme agent d'expansion. Les granules telles qu'obtenues présentent une densité apparente d'environ 0,15 à 0,2. Le produit tel qu'obtenu n'est donc pas constitué de saccharose pur et n'est pas compressible en raison de cette faible densité.

Un procédé de séchage par atomisation correspond au séchage d'une solution de saccharose par division en fines gouttelettes qui sont ensuite séchées sous un courant d'air chaud. Dans les documents de l'état de la technique, les procédés comprenant le séchage d'une solution de saccharose pure nécessitent une étape de pré-cristallisation de la solution. Ensuite, la suspension de petits cristaux est séchée par atomisation.

La demande internationale WO 02/06538 décrit un procédé d'atomisation de sucres, en particulier de saccharose, par co-atomisation d'une solution aqueuse de sucres ou d'une solution diluée de mélasse, avec un matériau solide, tel que du sucre en poudre ou du sucre glace. Les seuls produits décrits comme étant directement compressibles dans ce document sont obtenus par l'atomisation d'une solution de sucre inverti, de lactose, de maltodextrines ou d'un mélange de ces composés : ils ne sont donc pas constitués uniquement de saccharose car ils contiennent un agent liant.

Le brevet GB 1,240,691 concerne un procédé de production d'une composition de sucre solide non compressible qui comprend le séchage par atomisation d'un sirop de saccharose avec un taux de sucre inverti inférieur à 10% en masse et un taux en cendres inorganiques inférieur à 4% en masse. Par ailleurs, le pourcentage d'humidité résiduelle du produit obtenu est supérieur à 1 %, ce qui pose des problèmes de stockage du produit.

Le brevet GB 1,282,878 décrit un procédé d'atomisation par dispersion centrifuge simultanée d'une solution de saccharose concentrée et de particules de sucre solide. Ce procédé comprend notamment une étape d'introduction simultanée au niveau de la turbine de la solution de sucre et des cristaux de sucre. Le produit ainsi obtenu est une poudre constituée d'agglomérats de sucre microcristallisé, présentant un taux d'humidité résiduelle d'environ 0,5%, qui est un taux trop élevé pour éviter le mottage du produit au stockage.

De la même manière, d'autres brevets (GB 1 386 378, GB 1 386 379 et GB 1 387 062) décrivent des procédés d'atomisation d'un sirop de saccharose sur un support de poudre introduite ou recyclée. La quantité de solide mise en jeu est en général très importante (> 50% de la matière sèche du sirop entrant). Ces procédés s'apparentent à une granulation / séchage de poudre avec un sirop déposé par atomisation. Cette méthode conduit en général à une augmentation de la granulométrie et une réduction de la densité. Une étape de concassage est souvent nécessaire pour maintenir une densité et granulométrie stable pendant toute la production. Par ailleurs, toutes les poudres décrites ont une humidité résiduelle supérieure à 0,1%, ce qui ne permet pas de conserver les poudres de saccharose sans risque de mottage.

Dans le cadre d'un procédé de préparation par compactage, qui correspond à une granulation à sec, tous les ingrédients sont sous forme sèche et la composition pulvérulente résultante est agglomérée par pression, broyée et tamisée. L'addition de lubrifiants est nécessaire afin d'éviter les problèmes de collage. C'est le procédé utilisé notamment pour Nu-Tab® (Chr. Hansen).

Dans le cadre d'un procédé de préparation par microcristallisation, la solution de sucre est concentrée avec un taux de matières sèches supérieur à 90% et le mélange pâteux ainsi obtenu est refroidi sous battage mécanique. Une cristallisation-agglomération du produit permet de fabriquer une poudre compressible. C'est le procédé utilisé notamment pour Dipac® (Domino).

Les documents de l'état de la technique ne décrivent pas de procédé permettant d'obtenir une poudre de saccharose compressible et présentant une teneur en eau résiduelle appropriée pour sa conservation.

Il n'a donc jamais été décrit à ce jour une composition de saccharose adaptée à une compression directe et suffisamment stable à la conservation.

La présente invention a donc pour but de fournir une composition pulvérulente de saccharose compressible, ne contenant pas d'autres glucides ajoutés que ceux naturellement présents dans le saccharose, et présentant des propriétés adaptées au stockage.

La présente invention a également pour objet de proposer un procédé de préparation d'une composition pulvérulente de saccharose compressible, susceptible d'être mis en oeuvre à l'échelle industrielle.

La présente invention concerne une composition pulvérulente de saccharose compressible comprenant au moins 99,1% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules.

L'expression "composition pulvérulente de saccharose" désigne des microcristaux de saccharose agglomérés en particules, elles-mêmes pouvant être agglomérées entre elles. Selon un mode de réalisation préféré, la composition selon l'invention est constituée de sphères creuses.

L'expression "compressible" désigne l'aptitude d'une poudre à donner des comprimés de taille, de forme et de poids régulier en sortie d'une comprimeuse.

Un sucre directement compressible est un sucre sous forme pulvérulente qui ne nécessite qu'une étape de mélange des différents ingrédients (arômes, colorants, principes actifs, lubrifiants...) avant mise en oeuvre dans une comprimeuse permettant la fabrication de comprimés.

La composition pulvérulente de saccharose obtenue selon l'invention est directement compressible, elle peut donc être utilisée pour la préparation de comprimés. Cependant, cette propriété de la composition ne limite en aucun cas son utilisation à la fabrication de comprimés.

Le taux d'humidité résiduelle peut être mesuré par un dosage Karl Fischer.

Un taux d'humidité résiduelle supérieur à 0,1% n'est pas approprié pour un stockage de la composition. En effet, avec un tel taux, la composition n'est pas stable en raison de risques importants de mottage.

Selon un mode de réalisation préféré, la composition pulvérulente de saccharose compressible selon l'invention ne contient pas de liants, et, notamment, ne contient pas d'agent anti-cristallisant.

Selon les besoins, la composition pulvérulente selon l'invention peut également contenir des arômes ou des colorants, à raison de moins de 0,9% en poids par rapport au poids total de la composition.

De préférence, le sucre utilisé est un sucre issu de la fabrication ou du raffinage de sucres de canne ou de betterave, contenant à l'état sec, en poids déterminé par la méthode polarimétrique, au moins 99,1 % de saccharose.

La présente invention concerne une composition pulvérulente telle que définie ci-dessus, comprenant au moins 99,7% en poids de saccharose.

De préférence, on utilise les qualités sucre ou sucre blanc, sucre raffiné ou sucre blanc raffiné, qui contiennent au minimum 99,7% de saccharose et répondent par ailleurs à la directive CE n° 2001/111 (JOCE du 12 janvier 2002), en particulier une teneur en sucre inverti inférieure ou égale à 0,04% en poids.

Il est également possible d'utiliser le sucre mi-blanc qui contient au minimum 99,5% de saccharose et répond par ailleurs à la directive européenne CE n° 2001/111 (JOCE du 12 janvier 2002) et tout sucre contenant à l'état sec au moins 99,1% de saccharose et une teneur en sucre inverti inférieure ou égale à 0,1%.

Dans tous les cas, le sucre inverti présent en faible quantité est issu de la fabrication ou du raffinage du sucre et non post additionné par mélange ; à cette faible teneur, il ne peut donc pas jouer le rôle d'agent anti-cristallisant, qui permet de contrôler la cristallisation du saccharose pendant le séchage tel que défini dans la demande internationale WO 03/000936.

Comme autres exemples d'agents anti-cristallisants, on peut citer les maltodextrines, les monosaccharides, les oligosaccharides et les polyols.

Ainsi, aucun des composés naturellement présents autres que les 99,1%, et notamment les 99,7% de saccharose, ne sont en quantité suffisante pour jouer le rôle d'agent anti-cristallisant, même si parmi ces composés figurent des maltodextrines, des monosaccharides, des oligosaccharides et des polyols.

Selon un mode de réalisation avantageux, la composition pulvérulente selon l'invention ne contient pas de glucides ajoutés autres que le saccharose. Ainsi, cette composition ne contient pas d'autres glucides que ceux présents naturellement dans le saccharose utilisé.

Selon un mode de réalisation avantageux, la composition pulvérulente selon l'invention comprend uniquement du saccharose provenant de la fabrication ou du raffinage de sucres de canne ou de betterave.

Par ailleurs, les impuretés contenues dans le saccharose utilisé dans le cadre de la présente invention ne peuvent généralement pas être considérées comme des liants ou comme des agents anti-cristallisants.

De préférence, la présente invention concerne une composition pulvérulente telle que définie ci-dessus, comprenant 100% en poids de saccharose.

La présente invention concerne également une composition pulvérulente telle que définie ci-dessus, **caractérisée en ce que** la distribution granulométrique moyenne ou ouverture moyenne des particules creuses seules ou agglomérées varie d'environ 140 à environ 350 µm.

Les analyses granulométriques étant effectuées par tamisage, l'ouverture moyenne (OM) correspond à l'ouverture de tamis qui retient 50% en poids de l'échantillon.

Lorsque la taille des particules est supérieure à 350 µm, une telle composition n'est plus appropriée à la fabrication de comprimés de petite taille en raison de la difficulté de remplissage des matrices de la comprimeuse.

Lorsque la taille des particules est inférieure à 140 µm, les propriétés d'écoulement de la poudre sont dégradées, entraînant des variations sensibles de poids des comprimés.

La présente invention concerne une composition pulvérulente telle que définie ci-dessus, **caractérisée en ce que** le coefficient de variation desdites particules varie d'environ 35 à environ 55, notamment d'environ 40 à environ 50.

L'expression "coefficient de variation" désigne l'indicateur de dispersion autour de l'ouverture moyenne, c'est-à-dire de l'ouverture de tamis qui retient 50% en poids de l'échantillon. Plus exactement, le coefficient de variation est égal à l'écart-type de la répartition exprimée en pourcentage de l'ouverture moyenne.

Ces limites de dispersion granulométrique permettent d'obtenir une poudre compressible qui offre le meilleur compromis en termes d'écoulement de la poudre et d'aptitude au remplissage des matrices de la comprimeuse.

La présente invention concerne également une composition pulvérulente telle que définie ci-dessus, **caractérisée en ce qu'**elle présente une densité apparente variant d'environ 0,45 à environ 0,75.

L'expression "densité apparente" désigne la densité mesurée de la poudre ; il s'agit donc du rapport entre la masse de la poudre et le volume occupé par la poudre. Cette densité apparente s'oppose à la densité vraie de la poudre, qui correspond au rapport entre la masse de la poudre et le volume complètement rempli par ladite poudre sans volume d'air. On appelle degré de compactage d'une poudre le rapport entre la densité du comprimé et la densité vraie de la poudre.

On rappelle ici que la densité tassée et la densité non tassée sont définies selon la méthode décrite par la pharmacopée européenne (5ème édition - méthode de pharmacotechnie 2.9.15).

Pour évaluer l'aptitude à l'écoulement et au tassage d'une poudre, on peut également mesurer l'indice de Carr qui permet d'évaluer la coulabilité d'une poudre et correspond au rapport suivant :

$$\text{Indice de Carr} = 100 \times \frac{\text{Densité tassée} - \text{Densité non tassée}}{\text{Densité tassée}}$$

Ainsi, le tableau ci-après représente l'aptitude à l'écoulement et au tassage de poudres en fonction de l'indice de Carr :

| Indice de Carr | Aptitude à l'écoulement |
|---|---|
| 5 à 11% | Excellente |
| 12 à 17% | Bonne |
| 18 à 22% | Moyenne |
| 23 à 28% | Passable |
| 29 à 34% | Mauvaise |
| >35 % | Très mauvaise |

Dans le cadre de la présente invention, les indices de Carr des compositions pulvérulentes obtenues selon l'invention sont compris entre 8 et 17%.

Une composition pulvérulente préférée selon la présente invention est une composition telle que définie ci-dessus et présentant un temps d'écoulement inférieur à environ 15 secondes.

L'écoulement est tel que défini selon la méthode décrite par la pharmacopée européenne (5ème édition - méthode de pharmacotechnie 2.9.16).

Selon un mode de réalisation avantageux, la présente invention concerne une composition pulvérulente telle que définie ci-dessus, **caractérisée en ce qu'**elle présente un taux de cristallinité supérieur ou égal à 95%, notamment supérieur ou égal à 99%.

Le taux de cristallinité est évalué en mesurant par diffraction X la cristallinité de la composition pulvérulente comparée à la cristallinité de cristaux purs de saccharose.

La composition pulvérulente selon l'invention est **caractérisée en ce qu**'elle ne contient substantiellement pas de phase vitreuse ou amorphe.

La présente invention concerne également une composition pulvérulente telle que définie ci-dessus, **caractérisée en ce qu**'elle est susceptible d'être comprimée, la résistance de la poudre comprimée obtenue à partir de la composition pulvérulente étant supérieure à environ 1 MPa, ladite résistance étant mesurée pour une force de compression supérieure à environ 15 kN, et

la poudre comprimée répondant à une force d'éjection comprise d'environ 50 à environ 80 N, ladite force d'éjection étant mesurée pour une force de compression comprise d'environ 15 à environ 20 kN.

La résistance des comprimés obtenus est mesurée selon la méthode décrite dans la pharmacopée européenne, chapitre 2.9.8 (1997).

Le mécanisme utilisé pour comprimer la composition de sucre selon l'invention est, par exemple, une comprimeuse commercialisée par la société FROGERAIS, constituée de deux poinçons, le poinçon supérieur servant seul à la compression. A noter que la mesure de la résistance dépend en partie du type de comprimeuse utilisée ; ainsi, si les poinçons inférieurs et supérieurs servent simultanément à la compression, la résistance des comprimés obtenus sera plus élevée.

La force d'éjection est la force qu'il faut appliquer au poinçon inférieur pour libérer le comprimé.

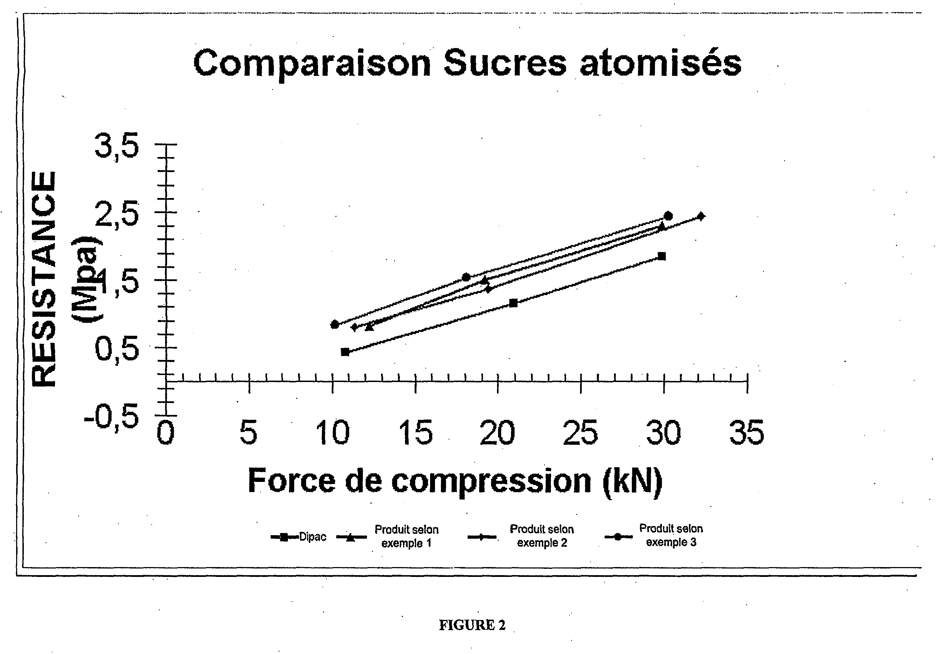

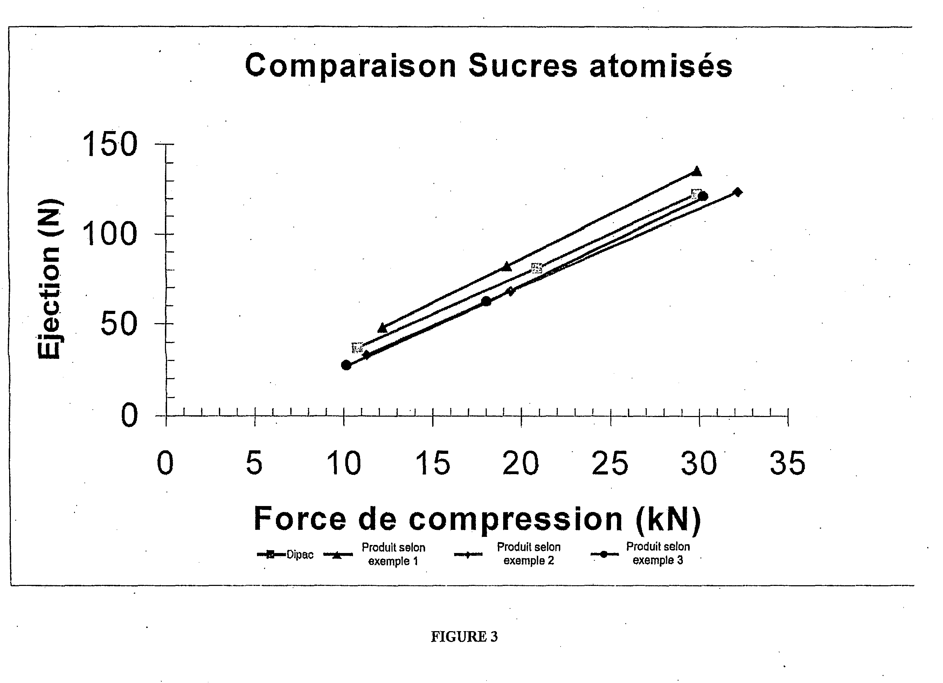

Les valeurs mesurées avec les poudres de l'invention sont indiquées sur des courbes et comparées sur les mêmes courbes aux valeurs obtenues avec la poudre de l'art antérieur (voir figures 2 et 3).

La présente invention concerne également un procédé continu de préparation d'une composition pulvérulente de saccharose compressible telle que définie ci-dessus, comprenant :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, et
- une éventuelle phase de refroidissement à l'air de la composition pulvérulente essentiellement cristallisée, pour obtenir la composition pulvérulente de saccharose compressible, ladite composition étant cristallisée et étant à une température inférieure à 30°C,

ladite phase de stabilisation et de cristallisation étant effectuée par mise en contact desdites particules vitreuses avec une partie de la composition pulvérulente essentiellement cristallisée recyclée ou avec une partie de la composition pulvérulente de saccharose compressible recyclée, le taux de recyclage de ladite composition pulvérulente essentiellement cristallisée ou de la composition pulvérulente de saccharose compressible variant d'environ 30 à environ 70% en poids de matières sèches de la solution de saccharose nébulisée.

L'expression "solution de saccharose nébulisée" désigne la division d'une solution de saccharose, notamment sous forme d'un sirop, en fines gouttelettes par une buse ou une turbine.

L'expression "tour d'atomisation" désigne l'installation qui permet de sécher une solution de saccharose nébulisée.

L'expression "phase de séchage" désigne la phase de déshydratation des gouttelettes de sirop de sucre par de l'air chaud.

L'expression "particules vitreuses de saccharose" désigne les gouttelettes de sirop déshydratées mais non encore cristallisées.

On introduit une solution de saccharose (solution de saccharose entrante ou solution de saccharose initiale), notamment sous forme de sirop dans une enceinte comportant des moyens d'atomisation et de séchage.

La première phase du procédé consiste en une phase de séchage de la solution de saccharose, notamment sous forme d'un sirop nébulisé sous forme de gouttelettes au moyen de buse ou de turbine. Ainsi, cette phase consiste à sécher les gouttes de saccharose formées par nébulisation, en particules solides avec un courant d'air chaud, dont la température d'entrée dans la tour d'atomisation (ou chambre de séchage) varie d'environ 140 à environ 180°C. L'air chaud ainsi utilisé pour le séchage est récupéré à la sortie de la tour d'atomisation sous forme humide.

L'expression "phase de stabilisation et de cristallisation" désigne le processus de cristallisation des particules de sucre vitreuses au contact de particules cristallines de saccharose.

L'expression "composition pulvérulente essentiellement cristallisée" désigne la composition pulvérulente de saccharose non encore refroidie.

La deuxième phase du procédé consiste à mettre en contact les particules vitreuses obtenues à l'issue de la première phase de séchage avec une partie de la composition pulvérulente essentiellement cristallisée recyclée ou avec une partie de la composition pulvérulente de saccharose compressible recyclée.

Cette deuxième phase permet notamment d'initier la cristallisation du saccharose.

Cette phase de mise en contact correspond à une collision entre les particules vitreuses instables obtenues à l'issue de la première phase de séchage et une partie de la composition pulvérulente essentiellement cristallisée recyclée ou une partie de la composition pulvérulente de saccharose compressible recyclée. La composition obtenue à l'issue de cette étape de mise en contact est essentiellement cristallisée.

En effet, ce mode de séchage en deux phases permet de retarder la cristallisation du sirop de saccharose en passant par un état amorphe (ou vitreux). Ce déclenchement de la cristallisation retardée par apport de saccharose essentiellement cristallisé et/ou saccharose compressible recyclé en dehors de la zone de nébulisation/séchage du sirop permet de générer des cristaux de taille réduite dans chaque particule creuse. En adoptant ce mode de fonctionnement, on obtient un arrangement des cristaux similaire à celui obtenu par exemple par le procédé décrit dans la demande internationale WO 03/000396, où l'ajout d'un agent anti-cristallisant est nécessaire.

Par la suite, l'expression "composition pulvérulente recyclée" désigne soit une partie de la composition pulvérulente essentiellement cristallisée recyclée, c'est-à-dire une partie de la composition pulvérulente obtenue avant la phase de refroidissement à l'air (composition non refroidie), soit une partie de la composition pulvérulente de saccharose compressible recyclée, c'est-à-dire une partie de la composition pulvérulente obtenue après refroidissement à l'air (composition refroidie à une température inférieure à 30°C).

Le procédé de l'invention est donc caractérisé par l'utilisation d'une composition pulvérulente recyclée telle que définie ci-dessus. Ainsi, on prélève un pourcentage de la composition pulvérulente essentiellement cristallisée recyclée ou de la composition pulvérulente de saccharose compressible, afin de la réutiliser dans la phase de stabilisation et de cristallisation, et donc mettre en contact ladite composition pulvérulente recyclée avec les particules vitreuses susmentionnées.

Le taux de recyclage de la composition pulvérulente ou de la poudre varie de 30 à 70% en poids de matières sèches de la solution de saccharose nébulisée.

Il est bien entendu possible d'obtenir la composition pulvérulente recherchée en travaillant avec un taux de recyclage plus important, notamment supérieur à 70%.

Par contre, si le taux de recyclage est inférieur à 30%, le nombre de collisions des particules vitreuses avec la poudre cristalline recyclée est insuffisant, ce qui occasionne le collage d'une partie de la poudre sur les parois de la tour d'atomisation et limite l'intérêt industriel du procédé.

Selon un mode de réalisation préféré, le procédé de préparation de l'invention est **caractérisé en ce que** la composition pulvérulente recyclée provient de la composition pulvérulente de saccharose compressible telle qu'obtenue à l'issue de la phase de refroidissement telle que définie ci-dessus.

Ainsi, dans ce mode de réalisation préféré, la composition pulvérulente recyclée utilisée lors de la mise en contact avec les particules vitreuses obtenues à l'issue de l'étape de séchage provient de la composition telle qu'obtenue à l'étape de refroidissement.

Si le taux d'humidité résiduelle est supérieur à 1%, le produit obtenu est un produit instable qui continue à perdre de l'eau, et cette eau peut se condenser sur n'importe quelle paroi de température inférieure à celle de l'eau. Ce phénomène entraîne en général un mottage.

Cette caractéristique est importante car elle va conditionner l'aptitude de la poudre à rester pulvérulente et à conserver sa fluidité. Pour une poudre à compression directe, ce paramètre va influer directement sur la régularité en poids et en densité des comprimés. Ainsi, une poudre trop humide présentera des agglomérats qui gêneront le bon déroulement du remplissage des matrices de la comprimeuse et induiront une augmentation rapide des forces d'éjection des comprimés et l'encrassement prématuré des équipements.

Le produit obtenu à l'issue de l'étape de mise en contact telle que définie ci-dessus est un produit non refroidi et instable. Ainsi, pour le stockage, il est avantageux, voire nécessaire de le soumettre à une étape de refroidissement. Sans cette étape de refroidissement, le produit obtenu est trop sensible aux variations climatiques et devient rapidement inutilisable.

A l'issue du procédé, on obtient la composition pulvérulente de saccharose compressible selon l'invention.

La présente invention concerne également un procédé continu de préparation d'une composition pulvérulente de saccharose compressible telle que définie ci-dessus, comprenant :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose,
- une phase de mise en contact desdites particules vitreuses telles qu'obtenues à l'issue de la phase précédente avec une poudre de cristaux de saccharose, ladite poudre étant présente dans une quantité correspondant à environ 30 à environ 70% en poids de matières sèches de la solution de saccharose nébulisée, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, et

- une éventuelle phase de refroidissement à l'air de la composition pulvérulente essentiellement cristallisée, pour obtenir la composition pulvérulente de saccharose compressible, ladite composition étant cristallisée et étant à une température inférieure à 30°C. Selon un mode de réalisation préféré, la poudre de cristaux de saccharose est une poudre recyclée provenant soit de la composition pulvérulente essentiellement cristallisée à une température comprise d'environ 50°C à environ 80°C et présentant un taux d'humidité résiduelle inférieur ou égal à 1%, telle qu'obtenue à l'issue de la phase de mise en contact telle que définie ci-dessus, soit de la composition pulvérulente de saccharose compressible telle qu'obtenue à l'issue de la phase de refroidissement.

De préférence, la poudre de cristaux de saccharose est une poudre recyclée provenant de la composition pulvérulente de saccharose compressible telle qu'obtenue à l'issue de la phase de refroidissement.

Le procédé de la présente invention est **caractérisé en ce qu**'il correspond à un procédé en continu, mis en oeuvre mis en oeuvre après une étape de démarrage jusqu'à l'obtention d'un régime stationnaire. Ainsi, il n'y a pas de montée en granulométrie et ce procédé permet d'obtenir une poudre présentant des caractéristiques constantes.

Le démarrage du procédé est effectué à partir de poudre de saccharose d'une production précédente jusqu'à obtention de la composition pulvérulente souhaitée.

En ce qui concerne le premier démarrage du procédé de l'invention, on utilise comme amorce de cristallisation, c'est-à-dire comme composition pulvérulente recyclée (ou poudre de cristaux de saccharose) telle que définie ci-dessus, un agent initial servant d'amorce de cristallisation. Un tel agent est un composé qui sert à initier la cristallisation lors du séchage, tant que l'on ne dispose pas encore de composition pulvérulente obtenue selon le procédé de l'invention.

Comme agent de cristallisation, on utilise notamment du sucre glace ou tout autre cristal de sucre.

Lorsqu'on a obtenu la composition pulvérulente en quantité suffisante pour être utilisée en partie comme amorce de cristallisation, c'est-à-dire comme composition pulvérulente recyclée (ou poudre de cristaux de saccharose) pour la phase de mise en contact, on supprime le sucre glace utilisé comme amorce de cristallisation.

La présente invention concerne également un procédé continu de préparation d'une composition pulvérulente de saccharose compressible telle que définie ci-dessus, comprenant :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose, et
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, ladite phase de stabilisation et de cristallisation étant effectuée par mise en contact desdites particules vitreuses avec une partie de la composition pulvérulente essentiellement cristallisée recyclée, le taux de recyclage de ladite composition pulvérulente essentiellement cristallisée variant d'environ 30 à environ 70% en poids de matières sèches de la solution de saccharose nébulisée.

Ce mode de réalisation comporte l'utilisation pour la phase de stabilisation et de cristallisation d'une partie de la composition pulvérulente essentiellement cristallisée recyclée.

La présente invention concerne un procédé continu de préparation d'une composition pulvérulente de saccharose compressible telle que définie ci-dessus, comprenant les étapes suivantes :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C, ladite phase de stabilisation et de cristallisation étant effectuée par mise en contact desdites particules vitreuses avec une partie de la composition pulvérulente essentiellement cristallisée recyclée ou avec une partie de la composition pulvérulente de saccharose compressible recyclée, le taux de recyclage de ladite composition pulvérulente essentiellement cristallisée ou de la composition pulvérulente de saccharose compressible variant d'environ 30 à environ 70% en poids de matières sèches de la solution de saccharose nébulisée.

Ce mode de réalisation comporte les trois phases, c'est-à-dire la phase de séchage, la phase de stabilisation et de cristallisation (collision des particules vitreuses avec la composition pulvérulente recyclée) et la phase de refroidissement.

Un mode de réalisation particulier du procédé de la présente invention comprend l'utilisation, comme composition pulvérulente recyclée, à la fois d'une partie de la composition pulvérulente essentiellement cristallisée et d'une partie de la composition pulvérulente compressible.

La présente invention concerne également un procédé continu de préparation d'une composition pulvérulente de saccharose compressible telle que définie ci-dessus, comprenant les étapes suivantes :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C, ladite phase de stabilisation et de cristallisation étant effectuée par mise en contact desdites particules vitreuses avec une partie de la composition pulvérulente essentiellement cristallisée recyclée, le taux de recyclage de ladite composition pulvérulente essentiellement cristallisée variant d'environ 30 à environ 70% en poids de matières sèches de la solution de saccharose nébulisée.

Ce mode de réalisation comporte les trois phases, c'est-à-dire la phase de séchage, la phase de stabilisation et de cristallisation (collision des particules vitreuses avec la composition pulvérulente recyclée) et la phase de refroidissement. Il est également caractérisé par l'utilisation d'une partie de la composition pulvérulente essentiellement cristallisée recyclée pour la phase de mise en contact avec les particules vitreuses.

La présente invention concerne également un procédé continu de préparation d'une composition pulvérulente de saccharose compressible telle que définie ci-dessus, comprenant les étapes suivantes :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C, ladite phase de stabilisation et de cristallisation étant effectuée par mise en contact desdites particules vitreuses avec une partie de la composition pulvérulente de saccharose compressible recyclée, le taux de recyclage de ladite composition pulvérulente de saccharose compressible variant d'environ 30 à environ 70% en poids de matières sèches de la solution de saccharose nébulisée.

Ce mode de réalisation comporte les trois phases, c'est-à-dire la phase de séchage, la phase de stabilisation et de cristallisation (collision des particules vitreuses avec la composition pulvérulente recyclée) et la phase de refroidissement. Il est également caractérisé par l'utilisation d'une partie de la composition pulvérulente de saccharose compressible recyclée pour la phase de mise en contact avec les particules vitreuses.

La présente invention concerne également un procédé continu de préparation d'une composition pulvérulente de saccharose compressible comprenant au moins 99,1% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules, ledit procédé comprenant :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, et
- une éventuelle phase de refroidissement à l'air de la composition pulvérulente essentiellement cristallisée, pour obtenir la composition pulvérulente de saccharose compressible, ladite composition étant cristallisée et étant à une température inférieure à 30°C,

ladite phase de stabilisation et de cristallisation étant effectuée par mise en contact desdites particules vitreuses avec une partie de la composition pulvérulente essentiellement cristallisée recyclée ou avec une partie de la composition pulvérulente de saccharose compressible recyclée, le taux de recyclage de ladite composition pulvérulente essentiellement cristallisée ou de la composition pulvérulente de saccharose compressible variant d'environ 30 à environ 70% en poids de matières sèches de la solution de saccharose nébulisée.

La présente invention concerne également le produit tel qu'obtenu par le procédé tel que défini ci-dessus.
La présente invention concerne également un procédé continu de préparation d'une composition pulvérulente de saccharose compressible comprenant au moins 99,1% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules, ledit procédé comprenant :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose, et
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, ladite phase de stabilisation et de cristallisation étant effectuée par mise en contact desdites particules vitreuses avec une partie de la composition pulvérulente essentiellement cristallisée recyclée, le taux de recyclage de ladite composition pulvérulente essentiellement cristallisée variant d'environ 30 à environ 70% en poids de matières sèches de la solution de saccharose nébulisée.

La présente invention concerne également le produit tel qu'obtenu par le procédé tel que défini ci-dessus.
La présente invention concerne également un procédé continu de préparation d'une composition pulvérulente de saccharose compressible comprenant au moins 99,1% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules, ledit procédé comprenant :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C, ladite phase de stabilisation et de cristallisation étant effectuée par mise en contact desdites particules vitreuses avec une partie de la composition pulvérulente essentiellement cristallisée recyclée ou avec une partie de la composition pulvérulente de saccharose compressible recyclée, le taux de recyclage de ladite composition pulvérulente essentiellement cristallisée ou de la composition pulvérulente de saccharose compressible variant d'environ 30 à environ 70% en poids de matières sèches de la solution de saccharose nébulisée.

La présente invention concerne également le produit tel qu'obtenu par le procédé tel que défini ci-dessus.
La présente invention concerne également un procédé continu de préparation d'une composition pulvérulente de saccharose compressible comprenant au moins 99,1% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules, ledit procédé comprenant :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C, ladite phase de stabilisation et de cristallisation étant effectuée par mise en contact desdites particules vitreuses avec une partie de la composition pulvérulente essentiellement cristallisée recyclée, le taux de recyclage de ladite composition pulvérulente essentiellement cristallisée variant d'environ 30 à environ 70% en poids de matières sèches de la solution de saccharose nébulisée.

La présente invention concerne également le produit tel qu'obtenu par le procédé tel que défini ci-dessus.
La présente invention concerne également un procédé continu de préparation d'une composition pulvérulente de saccharose compressible comprenant au moins 99,1% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules, ledit procédé comprenant :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C, ladite phase de stabilisation et de cristallisation étant effectuée par mise en contact desdites particules vitreuses avec une partie de la composition pulvérulente de saccharose compressible recyclée, le taux de recyclage de ladite composition pulvérulente de saccharose compressible variant d'environ 30 à environ 70% en poids de matières sèches de la solution de saccharose.

La présente invention concerne également le produit tel qu'obtenu par le procédé tel que défini ci-dessus.
La présente invention concerne également un procédé continu de préparation d'une composition pulvérulente de saccharose compressible comprenant au moins 99,7% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules, ledit procédé comprenant :

- une phase de séchage, par de l'air, d'une solution de saccharose nébulisée, dans une tour d'atomisation, la température de l'air ayant servi au séchage et sortant de ladite tour étant comprise d'environ 80 à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise d'environ 50°C à environ 80°C et présentant un taux d'humidité résiduelle inférieur ou égal à 1% en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C, ladite phase de stabilisation et de cristallisation étant effectuée par mise en contact desdites particules vitreuses avec une partie de la composition pulvérulente essentiellement cristallisée recyclée ou avec une partie de la composition pulvérulente de saccharose compressible recyclée, le taux de recyclage de ladite composition pulvérulente essentiellement cristallisée ou de la composition pulvérulente de saccharose compressible variant d'environ 30 à environ 70% en poids de matières sèches de la solution de saccharose nébulisée.

La présente invention concerne également le produit tel qu'obtenu par le procédé tel que défini ci-dessus.

Selon un mode préféré de réalisation, le procédé de l'invention est **caractérisé en ce que** la phase de séchage est effectuée aux alentours immédiats de la zone de nébulisation, notamment dans la partie haute de la tour d'atomisation.

L'expression "zone de nébulisation" désigne la zone où l'étape de nébulisation du procédé est effectuée, c'est-à-dire la zone où l'étape de séchage par de l'air chaud est effectuée.

L'expression "partie haute d'une tour d'atomisation" désigne la zone de nébulisation et de séchage du sirop de sucre.

Le procédé de l'invention est de préférence **caractérisé en ce que** la phase de mise en contact est effectuée dans la tour d'atomisation, dans des conditions telles que la composition recyclée est ajoutée au niveau d'une zone comprise entre le premier tiers et le deuxième tiers de ladite tour d'atomisation, et notamment au niveau de la zone médiane de ladite tour d'atomisation.

Selon encore un autre mode de réalisation préféré, la phase de mise en contact est effectuée en dehors de la zone de nébulisation et de séchage telle que définie ci-dessus.

La présente invention concerne également un procédé tel que défini ci-dessus, **caractérisé en ce que** la phase de refroidissement est effectuée à l'extérieur de la tour d'atomisation.

Selon un mode de réalisation avantageux, le procédé de l'invention est **caractérisé en ce que** la phase de refroidissement est effectuée dans des conditions permettant d'abaisser le taux d'humidité résiduelle de la composition pulvérulente essentiellement cristallisée et également de terminer la cristallisation de ladite composition afin d'obtenir la composition pulvérulente cristallisée de saccharose compressible.

On peut utiliser pour cette phase de refroidissement soit un lit fluidisé, un sécheur à tambour ou tout autre moyen qui permette le refroidissement de la composition pulvérulente.

La présente invention concerne également un procédé tel que défini ci-dessus, dans lequel la solution initiale de saccharose présente un taux de matières sèches d'environ 55 à 80% en poids.

Le taux de matières sèches d'un produit correspond au pourcentage d'extrait sec dudit produit.

Par ailleurs, à titre illustratif et non limitatif, le débit du sirop de saccharose introduit en vue d'être nébulisé varie d'environ quelques kg/h à environ plusieurs milliers de kg/h, notamment d'environ 500 kg à environ 2 000 kg/h, en fonction du dimensionnement de la tour d'atomisation et de ses capacités de séchage.

La présente invention concerne l'utilisation d'une composition pulvérulente de sucre compressible telle que définie ci-dessus, pour la préparation de comprimés, notamment pharmaceutiques.

La présente invention concerne l'utilisation d'une composition pulvérulente de sucre compressible telle que définie ci-dessus, pour la préparation de mélanges pulvérulents.

La présente invention concerne l'utilisation d'une composition pulvérulente de sucre compressible telle que définie ci-dessus, pour la préparation de formulations à dissolution rapide.

La présente invention concerne également l'utilisation d'une composition pulvérulente de sucre compressible telle que définie ci-dessus, en mélange avec au moins un autre produit, ledit autre produit étant sous forme soluble ou pulvérulente. De façon avantageuse, ledit autre produit est un principe actif, un édulcorant, un colorant, un arôme, une enzyme ou un agent tensio-actif.

Selon un mode de réalisation avantageux, le mélange ainsi obtenu comprenant la composition pulvérulente de sucre compressible telle que définie ci-dessus et ledit autre produit contient au plus 10% en poids dudit autre produit par rapport au poids du mélange.

La présente invention concerne également l'utilisation d'une composition pulvérulente de sucre compressible telle que définie ci-dessus, pour la fabrication de sphères de sucre, notamment par enrobage et/ou pelliculage et/ou dragéification.

Les sphères de sucre sont des particules sphériques ou quasi-sphériques constituées d'un noyau de saccharose sur lequel peuvent se juxtaposer des couches successives de glucides et/ou colloïdes et/ou de cires ou corps gras. La taille des particules obtenues peut varier de quelques dizaines de micromètres à quelques millimètres.

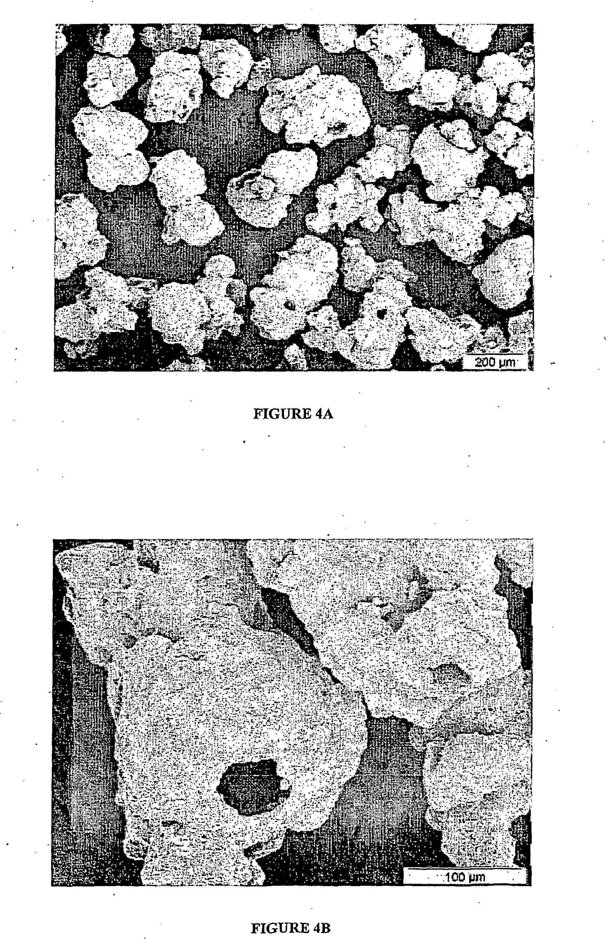

Compte tenu de la forme des particules et de leur état de surface (figures 4A et 4B), la composition pulvérulente de sucre compressible selon l'invention est parfaitement adaptée pour la fabrication de sphères de sucre. En effet, avec une forme presque sphérique, la poudre obtenue par atomisation permet, avec beaucoup plus de facilité, de former des sphères par pelliculage, enrobage ou dragéification. Grâce à son état de surface, cette poudre permet des dépôts de substances aussi bien hydrophiles (glucides, colloïdes, etc...) que hydrophobes (huiles, cires etc...).

## DESCRIPTION DES FIGURES

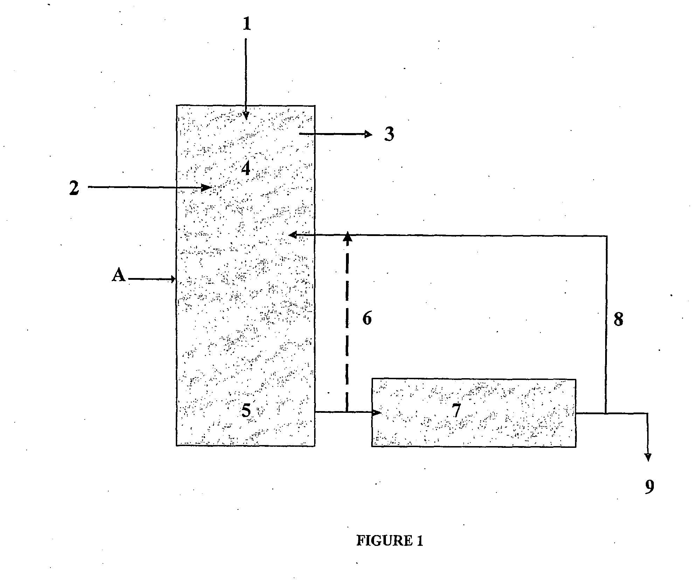

La Figure 1 représente un schéma général du procédé de l'invention.

La flèche 1 indique l'introduction du sirop de saccharose dans la tour d'atomisation (A). L'air de séchage (2) est introduit dans la zone de séchage (4) et l'air humide (3) est récupéré à une température d'environ 80 à 140°C. La zone (5) correspond à la zone de la tour d'atomisation où a lieu la phase de stabilisation et de cristallisation.

Une partie de la composition pulvérulente essentiellement cristallisée obtenue à l'issue de cette phase de stabilisation et de cristallisation peut par exemple être recyclée et réintroduite (6) dans la tour d'atomisation (A). La composition pulvérulente essentiellement cristallisée est soumise à une étape de refroidissement (7) pour obtenir une composition pulvérulente compressible refroidie (9). Une partie de cette composition pulvérulente compressible refroidie peut également être recyclée et réintroduite (8) dans la tour d'atomisation (A), étant entendu que le procédé de l'invention comporte nécessairement le recyclage de la composition pulvérulente, c'est-à-dire soit l'étape (6) soit l'étape (8).

La Figure 2 représente des courbes comparatives de la résistance des comprimés (en MPa) obtenus lors d'essais pilotes en fonction de la force de compression (en kN). La courbe avec les carrés noirs correspond au sucre Dipac ; la courbe avec les triangles noirs correspond à la composition pulvérulente de l'exemple 1 ; la courbe avec les losanges noirs correspond à la composition pulvérulente de l'exemple 2 et la courbe avec les ronds noirs correspond à la composition pulvérulente de l'exemple 3. L'examen des courbes de la Figure 2 font apparaître que les compositions pulvérulentes issues des exemples 1 à 3 selon l'invention présentent des résistances supérieures, pour une force de compression donnée, au produit commercial de référence (Dipac).

La Figure 3 représente des courbes comparatives de la force d'éjection en N à laquelle répondent des comprimés obtenus en fonction de la force de compression en kN. La courbe avec les carrés noirs correspond au sucre Dipac ; la courbe avec les triangles noirs correspond à la composition pulvérulente de l'exemple 1 ; la courbe avec les losanges noirs correspond à la composition pulvérulente de l'exemple 2 et la courbe avec les ronds noirs correspond à la composition pulvérulente de l'exemple 3.

On peut constater, d'après les courbes de la Figure 3, que les résultats obtenus avec les poudres de saccharose compressible de l'invention sont comparables à ceux obtenus pour le produit commercial Dipac®, en termes de force d'éjection pour une force de compression donnée.

Les courbes des Figures 2 et 3 sont à analyser en parallèle car la force d'éjection supportable par un comprimé sans risque de casse ne peut être dissociée de la résistance de ce comprimé. Ainsi, les comprimés obtenus élaborés à partir de compositions pulvérulentes de saccharose selon l'invention présentent des couples résistance/force d'éjection comparables à ceux obtenus à partir du produit commercial Dipac®.

Les Figures 4A et 4B représentent des images de la composition pulvérulente directement compressible sous un microscope électronique à balayage.

La Figure 4A montre que la composition pulvérulente obtenue est composée de particules creuses seules ou associées entre elles pour former des granules.

La Figure 4B représente un détail d'une particule creuse d'environ 200 $\mu$m, constituée de microcristaux agglomérés et associée à d'autres particules de dimensions variables.

## EXEMPLES

### Exemple 1

Une solution de saccharose (65% de matières sèches) est nébulisée à 102 bars dans une chambre de séchage sous courant d'air chaud. La température de l'air de séchage est régulée en fonction de la consigne de la température de l'air humide de sortie. La poudre sortant de la chambre de séchage et refroidie est recyclée partiellement (le taux de recyclage est de 40% par rapport à la matière sèche du sirop entrant) vers la partie basse de la chambre, en dehors de la zone de nébulisation de la solution.

### Paramètres de fonctionnement :

Entrée

| Sirop | 65 % matière sèche (MS) | 86°C |
|---|---|---|
| Débit sirop (65% MS) | 660 kg/heure | |

(suite)

| Sirop | 65 % matière sèche (MS) | 86°C |
|---|---|---|
| Température air (séchage) | 145°C | |
| Taux de recyclage | 40% (172 kg) | |

Sortie

| | |
|---|---|
| Température air | 90°C |
| Température de la composition pulvérulente à la sortie de la chambre de séchage (avant refroidissement) | 70°C |
| Humidité de la composition pulvérulente à la sortie de la chambre de séchage (avant refroidissement) | 0,85% MS |
| Température de la composition pulvérulente refroidie | 30°C |
| Humidité de la composition pulvérulente refroidie | <0,1% MS |

Caractéristiques de la poudre séchée

| OM ($\mu$m) | CV (%) | Ecoulement secondes/100g | Densité non tassée (g/ml) | Densité tassée (g/ml) | Indice de Carr % |
|---|---|---|---|---|---|
| 147 | 50 | 12,7 | 0,6 | 0,72 | 17 |

**Exemple 2**

Une solution de saccharose (65% de matières sèches) est nébulisée à 210 bars dans une chambre de séchage sous courant d'air chaud. La température de l'air de séchage est régulée en fonction de la consigne de la température de l'air humide de sortie. La poudre sortant de la chambre de séchage et refroidie est recyclée partiellement (le taux de recyclage est de 40% par rapport au taux de matières sèches du sirop entrant) vers la partie basse de la chambre, en dehors de la zone de nébulisation de la solution.

**Paramètres de fonctionnement :**

Entrée

| Sirop | 65% matière sèche (MS) | 76°C |
|---|---|---|
| Débit sirop (65% MS) | 1923 kg/heure | |
| Température air (séchage) | 180°C | |
| Taux de recyclage | 40% (500 kg) | |

Sortie

| | |
|---|---|
| Température air | 120°C |
| Température de la composition pulvérulente à la sortie de la chambre de séchage (avant refroidissement) | 80°C |
| Humidité de la composition pulvérulente à la sortie de la chambre de séchage (avant refroidissement) | MS 0,95% MS |
| Température de la composition pulvérulente refroidie | 30°C |
| Humidité de la composition pulvérulente refroidie | <0,1% MS |

Caractéristiques de la poudre séchée

| OM (μm) | CV (%) | Ecoulement secondes/100g | Densité non tassée (g/ml) | Densité tassée (g/ml) | Indice de Carr % |
|---|---|---|---|---|---|
| 174 | 43 | 12,7 | 0,53 | 0,63 | 16 |

La composition pulvérulente de saccharose obtenue selon l'invention est aussi performante qu'une poudre obtenue formulée avec des liants (voir Figures 2 et 3).

**Exemple 3**

Une solution de saccharose (55% de matières sèches) est nébulisée à 210 bars dans une chambre de séchage sous courant d'air chaud. La température de l'air de séchage est régulée en fonction de la consigne de la température de l'air humide de sortie. La poudre sortant de la chambre de séchage et non refroidie est recyclée partiellement (35% par rapport au taux de matières sèches du sirop entrant) vers la partie basse de la chambre, en dehors de la zone de nébulisation de la solution.

**Paramètres de fonctionnement :**

Entrée

| | | |
|---|---|---|
| Sirop | 55% matière sèche (MS) | 70°C |
| Débit sirop (55% MS) | 2000 kg/heure | |
| Température air (séchage) | 195°C | |
| Taux de recyclage | 35% (385 kg) | |

Sortie

| | |
|---|---|
| Température air | 105°C |
| Température de la composition pulvérulente à la sortie de la chambre de séchage (avant refroidissement) | 72°C |
| Humidité de la composition pulvérulente à la sortie de la chambre de séchage (avant refroidissement) | 0,9% MS |
| Température de la composition pulvérulente refroidie | 28°C |
| Humidité de la composition pulvérulente refroidie | <0,1% MS |

Caractéristiques de la poudre séchée

| OM (μm) | CV (%) | Ecoulement secondes/100g | Densité non tassée (g/ml) | Densité tassée (g/ml) | Indice de Carr % |
|---|---|---|---|---|---|
| 164 | 45 | 13 0,51 | | 0,61 | 16 |

La composition pulvérulente de saccharose obtenue selon l'invention est aussi performante qu'une poudre obtenue formulée avec des liants (voir Figures 2 et 3).

Le recyclage de la poudre essentiellement cristallisée permet d'obtenir un produit tout à fait comparable aux produits obtenus selon les exemples 1 et 2.

## Composition et Protocole d'obtention des comprimés.

Les comprimés ont été obtenus sur une machine alternative Frogerais OA, dont le format de la chambre de compression est de 11,28 mm de section. La vitesse de la machine est de 3350 cps/heure.

Avant la phase de compression, les compositions pulvérulentes sont mélangées à 0,5 (Dipac) ou 0,75% (poudres atomisées selon l'invention) de stéarate de magnésium pendant 5 minutes dans un mélangeur à mouvement tridimensionnel aléatoire. Les comprimés ronds et plats ont été confectionnés avec des forces de compression de 10, 20 et 30 kN.

## Exemple 4

Dans cet exemple, un colorant est choisi pour simuler un ingrédient (principe actif, arôme, enzyme, tensioactifs etc....). Le colorant, de distribution granulométrique choisie, est mélangé au sucre séché par atomisation (composition de l'invention). L'homogénéité du mélange est évaluée en fonction du temps de mélange, du taux d'incorporation et de la taille des particules de colorant. Par sa morphologie et son état de surface, le sucre séché par atomisation permet d'obtenir des mélanges parfaitement homogènes. En effet, même avec un mélangeur à pale, les mélanges obtenus dans les conditions citées ci-dessous ont un dosage homogène, dont l'écart type est toujours inférieur à 2% (moyenne sur 10 échantillons prélevés).

### Homogénéité du mélange en fonction des temps de mélange

| Taille des particules du colorant | < 80 μm | < 80 μm | < 80 μm |
|---|---|---|---|
| % incorporation | 0,5% | 0,5% | 0,5% |
| **Temps mélange à sec (minutes)** | **3** | **20** | **40** |
| Ecart-type sur 10 mesures / % d'incorporation cible du colorant | 1% | 1,5% | 1,5% |

### Homogénéité du mélange en fonction du taux d'incorporation

| Taille des particules du colorant | < 80 μm | < 80 μm | < 80 μm | < 80 μm |
|---|---|---|---|---|
| **% incorporation** | **0,01%** | **0,075%** | **0,5%** | **5%** |
| Temps mélange à sec (minutes) | 3 | 3 | 3 | 3 |
| Ecart type sur 10 mesures / % d'incorporation cible du colorant | 1,3% | 0,9% | 0,9% | 1,9% |

**Homogénéité du mélange en fonction de la taille des particules de colorant**

| Taille des particules du colorant | < 50 μm | < 80 μm | < 315 μm |
|---|---|---|---|
| % incorporation | 0,5% | 0,5% | 0,5% |
| Temps mélange à sec (minutes) | 3 | 3 | 3 |
| Ecart type sur 10 mesure / % d'incorporation cible du colorant | 1% | 0,5% | 1,3% |

D'après ces résultats, on constate que tous les mélanges obtenus sont des mélanges homogènes car aucun redémélange n'est observé (mélange stable).

Méthode de dosage du colorant

Le dosage du colorant dans le mélange est réalisé par mesure d'absorbance sur un spectromètre UV/ visible UNICAM UV4 à la longueur d'onde de 500 nm avec une cellule de 10 mm de longueur. Le pourcentage de colorant est calculé par rapport aux mesures réalisées avec des solutions standard.

**Revendications**

1. Composition pulvérulente de saccharose compressible comprenant au moins 99,1 % en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules.

2. Composition pulvérulente selon la revendication 1, comprenant au moins 99,7% en poids de saccharose.

3. Composition pulvérulente selon la revendication 1 ou 2, **caractérisée en ce que** la distribution granulométrique moyenne des particules creuses seules ou agglomérées varie de 140 à 350 μm.

4. Composition pulvérulente selon la revendication 3, **caractérisée en ce que** le coefficient de variation desdites particules varie de 35 à 55, notamment de 40 à 50.

5. Composition pulvérulente selon l'une quelconque des revendications 1 à 4, **caractérisée en ce qu'**elle présente une densité apparente variant de 0,45 à 0,75.

6. Composition pulvérulente selon l'une quelconque des revendications 1 à 5, **caractérisée en ce qu'**elle présente un temps d'écoulement inférieur à environ 15 secondes.

7. Composition pulvérulente selon l'une quelconque des revendications 1 à 6, **caractérisée en ce qu'**elle est susceptible d'être comprimée, la résistance de la poudre comprimée obtenue à partir de la composition pulvérulente étant supérieure à environ 1 MPa, ladite résistance étant mesurée pour une force de compression supérieure à environ 15 kN, et la poudre comprimée répondant à une force d'éjection comprise d'environ 50 à environ 80 N, ladite force d'éjection étant mesurée pour une force de compression comprise de 15 à 20 kN.

8. Procédé continu de préparation d'une composition pulvérulente de saccharose compressible telle que définie dans l'une quelconque des revendications 1 à 7, comprenant :

   - une phase de séchage, par de l'air, d'une solution de saccharose nébulisée, dans une tour d'atomisation, la température de l'air ayant servi au séchage et sortant de ladite tour étant comprise de 80°C à 140°C, pour obtenir des particules vitreuses de saccharose,
   - une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1% en poids par rapport au poids total de ladite composition, et

- une éventuelle phase de refroidissement à l'air de la composition pulvérulente essentiellement cristallisée, pour obtenir la composition pulvérulente de saccharose compressible, ladite composition étant cristallisée et étant à une température inférieure à 30°C,

ladite phase de stabilisation et de cristallisation étant effectuée par mise en contact desdites particules vitreuses avec une partie de la composition pulvérulente essentiellement cristallisée recyclée ou avec une partie de la composition pulvérulente de saccharose compressible recyclée, le taux de recyclage de ladite composition pulvérulente essentiellement cristallisé ou de la composition pulvérulente de saccharose compressible variant de 30% à 70% en poids de matières sèches de la solution de saccharose nébulisée.

9. Procédé continu de préparation d'une composition pulvérulente de saccharose compressible telle que définie dans l'une quelconque des revendications 1 à 7, comprenant :

- une phase de séchage, par de l'air, d'une solution de saccharose nébulisée, dans une tour d'atomisation, la température de l'air ayant servi au séchage et sortant de ladite tour étant comprise de 80°C à 140°C, pour obtenir des particules vitreuses de saccharose, et
- une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1% en poids par rapport au poids total de ladite composition,

ladite phase de stabilisation et de cristallisation étant effectuée par mise en contact desdites particules vitreuses avec une partie de la composition pulvérulente essentiellement cristallisée recyclée, le taux de recyclage de ladite composition pulvérulente essentiellement cristallisée variant de 30% à 70% en poids de matières sèches de la solution de saccharose nébulisée.

10. Procédé continu de préparation d'une composition pulvérulente de saccharose compressible telle que définie dans l'une quelconque des revendications 1 à 7, comprenant les étapes suivantes :

- une phase de séchage, par de l'air, d'une solution de saccharose nébulisée, dans une tour d'atomisation, la température de l'air ayant servi au séchage et sortant de ladite tour étant comprise de 80°C à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1% en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C,

ladite phase de stabilisation et de cristallisation étant effectuée par mise en contact desdites particules vitreuses avec une partie de la composition pulvérulente essentiellement cristallisée recyclée ou avec une partie de la composition pulvérulente de saccharose compressible recyclée, le taux de recyclage de ladite composition pulvérulente essentiellement cristallisée ou de la composition pulvérulente de saccharose compressible variant de 30% à 70% en poids de matières sèches de la solution de saccharose nébulisée.

11. Procédé continu de préparation d'une composition pulvérulente de saccharose compressible telle que définie dans l'une quelconque des revendications 1 à 7, comprenant les étapes suivantes :

- une phase de séchage, par de l'air, d'une solution de saccharose nébulisée, dans une tour d'atomisation, la température de l'air ayant servi au séchage et sortant de ladite tour étant comprise de 80°C à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1% en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C,

ladite phase de stabilisation et de cristallisation étant effectuée par mise en contact desdites particules vitreuses avec une partie de la composition pulvérulente essentiellement cristallisée recyclée, le taux de recyclage de ladite composition pulvérulente essentiellement cristallisée variant de 30% à 70% en poids de matières sèches de la solution de saccharose nébulisée.

12. Procédé continu de préparation d'une composition pulvérulente de saccharose compressible telle que définie dans l'une quelconque des revendications 1 à 7, comprenant les étapes suivantes :

- une phase de séchage, par de l'air, d'une solution de saccharose nébulisée, dans une tour d'atomisation, la température de l'air ayant servi au séchage et sortant de ladite tour étant comprise de 80°C à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1 % en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C,
ladite phase de stabilisation et de cristallisation étant effectuée par mise en contact desdites particules vitreuses avec une partie de la composition pulvérulente de saccharose compressible recyclée, le taux de recyclage de ladite composition pulvérulente de saccharose compressible variant de 30% à 70% en poids de matières sèches de la solution de saccharose nébulisée.

13. Procédé selon l'une quelconque des revendications 8 à 12, dans lequel la phase de séchage est effectuée aux alentours immédiats de la zone de nébulisation, notamment dans la partie haute de la tour d'atomisation.

14. Procédé selon l'une quelconque des revendications 8 à 13, dans lequel la phase de mise en contact est effectuée dans la tour d'atomisation, dans des conditions telles que la composition recyclée est ajoutée au niveau d'une zone comprise entre le premier tiers et le deuxième tiers de ladite tour d'atomisation, et notamment au niveau de la zone médiane de ladite tour d'atomisation.

15. Procédé selon l'une quelconque des revendications 8 à 14, **caractérisé en ce que** la phase de refroidissement est effectuée à l'extérieur de la tour d'atomisation.

16. Procédé selon l'une quelconque des revendications 8 à 15, **caractérisé en ce que** la phase de refroidissement est effectuée dans des conditions permettant d'abaisser le taux d'humidité résiduelle de la composition pulvérulente essentiellement cristallisée et également de terminer la cristallisation de ladite composition afin d'obtenir la composition pulvérulente cristallisée de saccharose compressible.

17. Procédé selon l'une quelconque des revendications 8 à 16, dans lequel la solution initiale de saccharose présente un taux de matières sèches de 55% à 80% en poids.

18. Procédé continu de préparation d'une composition pulvérulente de saccharose compressible comprenant au moins 99,1% en poids de saccharose, présentant un taux d'humidité résiduelle inférieur ou égal à 0, 1 % en poids par rapport au poids total de ladite composition, ladite composition étant constituée de particules creuses, éventuellement agglomérées entre elles sous forme de granules, ledit procédé comprenant :

- une phase de séchage, par de l'air, d'une solution de saccharose nébulisée, dans une tour d'atomisation, la température de l'air ayant servi au séchage et sortant de ladite tour étant comprise de 80°C à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1% en poids par rapport au poids total de ladite composition, et
- une éventuelle phase de refroidissement à l'air de la composition pulvérulente essentiellement cristallisée, pour obtenir la composition pulvérulente de saccharose compressible, ladite composition étant cristallisée et étant à une température inférieure à 30°C,
ladite phase de stabilisation et de cristallisation étant effectuée par mise en contact desdites particules vitreuses avec une partie de la composition pulvérulente essentiellement cristallisée recyclée ou avec une partie de la composition pulvérulente de saccharose compressible recyclée, le taux de recyclage de ladite composition pulvérulente essentiellement cristallisée ou de la composition pulvérulente de saccharose compressible variant de 30 à 70% en poids de matières sèches de la solution de saccharose nébulisée.

**19.** Procédé continu de préparation d'une composition pulvérulente de saccharose compressible comprenant au moins 99,1% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules, ledit procédé comprenant :

- une phase de séchage, par de l'air, d'une solution de saccharose nébulisée, dans une tour d'atomisation, la température de l'air ayant servi au séchage et sortant de ladite tour étant comprise de 80°C à 140°C, pour obtenir des particules vitreuses de saccharose, et
- une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1% en poids par rapport au poids total de ladite composition,
ladite phase de stabilisation et de cristallisation étant effectuée par mise en contact desdites particules vitreuses avec une partie de la composition pulvérulente essentiellement cristallisée recyclée, le taux de recyclage de ladite composition pulvérulente essentiellement cristallisée variant de 30% à 70% en poids de matières sèches de la solution de saccharose nébulisée.

**20.** Procédé continu de préparation d'une composition pulvérulente de saccharose compressible comprenant au moins 99,1% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules, ledit procédé comprenant :

- une phase de séchage, par de l'air, d'une solution de saccharose nébulisée, dans une tour d'atomisation, la température de l'air ayant servi au séchage et sortant de ladite tour étant comprise 80°C à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1% en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C,
ladite phase de stabilisation et de cristallisation étant effectuée par mise en contact desdites particules vitreuses avec une partie de la composition pulvérulente essentiellement cristallisée recyclée ou avec une partie de la composition pulvérulente de saccharose compressible recyclée, le taux de recyclage de ladite composition pulvérulente essentiellement cristallisée ou de la composition pulvérulente de saccharose compressible variant de 30% à 70% en poids de matières sèches de la solution de saccharose nébulisée.

**21.** Procédé continu de préparation d'une composition pulvérulente de saccharose compressible comprenant au moins 99,1% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules, ledit procédé comprenant :

- une phase de séchage, par de l'air, d'une solution de saccharose nébulisée, dans une tour d'atomisation, la température de l'air ayant servi au séchage et sortant de ladite tour étant comprise de 80°C à 140°C, pour obtenir des particules vitreuses de saccharose,
- une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1 % en poids par rapport au poids total de ladite composition, et
- une phase de refroidissement à l'air de la composition pulvérulente essentiellement cristallisée, pour obtenir la composition pulvérulente de saccharose compressible, ladite composition étant cristallisée et étant à une température inférieure à 30°C,
ladite phase de stabilisation et de cristallisation étant effectuée par mise en contact desdites particules vitreuses avec une partie de la composition pulvérulente essentiellement cristallisée recyclée, le taux de recyclage de ladite composition pulvérulente essentiellement cristallisée variant de 30 à 70% en poids de matières sèches de la solution de saccharose nébulisée.

22. Procédé continu de préparation d'une composition pulvérulente de saccharose compressible comprenant au moins 99,1% en poids de saccharose, présentant un taux d'humidité résiduelle inférieur ou égal à 0,1 % en poids par rapport au poids total de ladite composition, ladite composition étant constituée de particules creuses, éventuellement agglomérées entre elles sous forme de granules, ledit procédé comprenant :

    - une phase de séchage, par de l'air, d'une solution de saccharose nébulisée, dans une tour d'atomisation, la température de l'air ayant servi au séchage et sortant de ladite tour étant comprise de 80°C à 140°C, pour obtenir des particules vitreuses de saccharose,
    - une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1% en poids par rapport au poids total de ladite composition, et
    - une phase de refroidissement à l'air de la composition pulvérulente essentiellement cristallisée, pour obtenir la composition pulvérulente de saccharose compressible, ladite composition étant cristallisée et étant à une température inférieure à 30°C,
    ladite phase de stabilisation et de cristallisation étant effectuée par mise en contact desdites particules vitreuses avec une partie de la composition pulvérulente de saccharose compressible recyclée, le taux de recyclage de ladite composition pulvérulente de saccharose compressible variant de 30% à 70% en poids de matières sèches de la solution de saccharose nébulisée.

23. Procédé continu de préparation d'une composition pulvérulente de saccharose compressible comprenant 100% en poids de saccharose, présentant un taux d'humidité résiduelle inférieur ou égal à 0,1% en poids par rapport au poids total de ladite composition, ladite composition étant constituée de particules creuses, éventuellement agglomérées entre elles sous forme de granules, ledit procédé comprenant :

    - une phase de séchage, par de l'air, d'une solution de saccharose nébulisée, dans une tour d'atomisation, la température de l'air ayant servi au séchage et sortant de ladite tour étant comprise de 80°C à 140°C, pour obtenir des particules vitreuses de saccharose;
    - une phase de stabilisation et de cristallisation des particules vitreuses obtenues à l'issue de la phase de séchage, pour obtenir une composition pulvérulente essentiellement cristallisée à une température comprise de 50°C à 80°C et présentant un taux d'humidité résiduelle inférieur ou égal à 1% en poids par rapport au poids total de ladite composition, et
    - une phase de refroidissement à l'air de la composition pulvérulente essentiellement cristallisée, pour obtenir la composition pulvérulente de saccharose compressible, ladite composition étant cristallisée et étant à une température inférieure à 30°C,
    ladite phase de stabilisation et de cristallisation étant effectuée par mise en contact desdites particules vitreuses avec une partie de la composition pulvérulente essentiellement cristallisée recyclée ou avec une partie de la composition pulvérulente de saccharose compressible recyclée, le taux de recyclage de ladite composition pulvérulente essentiellement cristallisée ou de la composition pulvérulente de saccharose compressible variant de 30% à 70% en poids de matières sèches de la solution de saccharose nébulisée.

24. Composition pulvérulente de sucre compressible telle qu'obtenue par le procédé selon l'une des revendications 18 à 23.

25. Utilisation d'une composition pulvérulente de sucre compressible selon l'une des revendications 1 à 7 ou 24, pour la préparation de comprimés, notamment pharmaceutiques.

26. Utilisation d'une composition pulvérulente de sucre compressible selon l'une des revendications 1 à 7 ou 24, pour la préparation de mélanges pulvérulents.

27. Utilisation d'une composition pulvérulente de sucre compressible selon l'une des revendications 1 à 7 ou 24, pour la préparation de formulations à dissolution rapide.

28. Utilisation d'une composition pulvérulente de sucre compressible selon l'une des revendications 1 à 7 ou 24, en mélange avec au moins un autre produit, ledit autre produit étant sous forme soluble ou pulvérulente, et étant choisi parmi les produits suivants : principe actif, édulcorant, colorant, arôme, enzyme ou agent tensio-actif.

29. Utilisation d'une composition pulvérulente de sucre compressible selon l'une des revendications 1 à 7 ou 24, pour la fabrication de sphères de sucre, notamment par enrobage et/ou pelliculage et/ou dragéification.

**Claims**

1. Compressible, powdery saccharose composition comprising at least 99.1% by weight of saccharose, exhibiting a residual moisture content of less than 0.1 % by weight, based on the total weight of said composition, said composition consisting of hollow particles, optionally agglomerated together in the form of granules.

2. Powdery composition according to claim 1, comprising at least 99.7% by weight of saccharose.

3. Powdery composition according to claim 1 or 2, **characterised in that** the average granulometric distribution of the hollow particles alone or agglomerated varies from 140 to 350 $\mu$m.

4. Powdery composition according to claim 3, **characterised in that** the coefficient of variation of said particles varies from 35 to 55, particularly from 40 to 50.

5. Powdery composition according to any one of claims 1 to 4, **characterised in that** its bulk density is in the range of 0.45 to 0.75.

6. Powdery composition according to any one of claims 1 to 5, **characterised in that** its flow time is less than about 15 seconds.

7. Powdery composition according to any one of claims 1 to 6, **characterised in that** it is able to be compressed, the strength of the compressed powder obtained from the powdery composition being greater than about 1 MPa, said strength being measured for a compression force greater than about 15 kN, and the compressed powder meeting an ejection force comprised between about 50 to about 80 N, said ejection force being measured for a compression force comprised between 15 to 20 kN.

8. Continuous process for manufacturing a compressible, powdery saccharose composition such as defined in any one of claims 1 to 7, comprising:

   - a drying step, by air, of a nebulised saccharose solution in an atomisation tower, the temperature of the air after drying and exiting said tower being comprised between 80 °C to 140 °C, for obtaining the glassy particles of saccharose,
   - a stabilisation and crystallisation step of the glassy particles obtained from the drying step to obtain an essentially crystallised powdery composition at a temperature comprised between 50 °C to 80 °C and exhibiting a residual moisture content of less than or equal to 1% by weight based on the total weight of the composition, and
   - an optional cooling step in air of the essentially crystallised powdery composition, to obtain the compressible, powdery saccharose composition, said composition being crystallised and being at a temperature of less than 30 °C,
   said stabilisation and crystallisation step being carried out by contacting said glassy particles with part of the recycled, essentially crystallised powdery composition or with part of the recycled, compressible, powdery saccharose composition, the amount of recycle of said essentially crystallised powdery composition or of the compressible, powdery saccharose composition ranging from 30 to 70% by weight of dry matter of the nebulised saccharose solution.

9. Continuous process for manufacturing a compressible, powdery saccharose composition such as defined in any one of claims 1 to 7, comprising:

   - a drying step, by air, of a nebulised saccharose solution in an atomisation tower, the temperature of the air after the drying and exiting said tower being comprised between 80°C to 140 °C, for obtaining the glassy particles of saccharose, and
   - a stabilisation and crystallisation step of the glassy particles obtained from the drying step to obtain an essentially crystallised powdery composition at a temperature comprised between 50 °C to 80 °C and exhibiting a residual moisture content of less than or equal to 1% by weight based on the total weight of said composition,
said stabilisation and crystallisation step being carried out by contacting said glassy particles with part of the recycled, essentially crystallised powdery composition, the amount of recycle of said essentially crystallised powdery composition varying from 30% to 70% by weight of dry matter of the nebulised saccharose solution.

10. Continuous process for manufacturing a compressible, powdery saccharose composition such as defined in any one of claims 1 to 7, comprising the following steps:

- a drying step, by air, of a nebulised saccharose solution in an atomisation tower, the temperature of the air used for drying and exiting said tower being comprised between 80°C to 140 °C, for obtaining the glassy particles of saccharose,
- a stabilisation and crystallisation step of the glassy particles obtained from the drying step to obtain an essentially crystallised powdery composition at a temperature comprised between 50 °C to 80 °C and exhibiting a residual moisture content of less than or equal to 1% by weight based on the total weight of said composition, and
- a cooling step in air of the essentially crystallised powdery composition, to obtain the compressible, powdery saccharose composition, said composition being crystallised and being at a temperature of less than 30 °C, said stabilisation and crystallisation step being carried out by contacting said glassy particles with part of the recycled, essentially crystallised powdery composition or with part of the recycled, compressible, powdery saccharose composition, the amount of recycle of said essentially crystallised powdery composition or of the compressible, powdery saccharose composition varying from about 30 to about 70% by weight of dry matter of the nebulised saccharose solution.

11. Continuous process for manufacturing a compressible, powdery saccharose composition such as defined in any one of claims 1 to 7, comprising the following steps:

- a drying step, by air, of a nebulised saccharose solution in an atomisation tower, the temperature of the air after drying and exiting said tower being comprised between 80 °C to 140 °C, for obtaining the glassy particles of saccharose,
- a stabilisation and crystallisation step of the glassy particles obtained from the drying step to obtain an essentially crystallised powdery composition at a temperature comprised between 50 °C to 80 °C and exhibiting a residual moisture content of less than or equal to 1% by weight based on the total weight of said composition, and
- a cooling step in air of the essentially crystallised powdery composition to obtain the compressible, powdery saccharose composition, said composition being crystallised and being at a temperature of less than 30 °C, said stabilisation and crystallisation step being carried out by contacting said glassy particles with part of the recycled, essentially crystallised powdery composition, the amount of recycle of said essentially crystallised powdery composition varying from 30 to 70% by weight of dry matter of the nebulised saccharose solution.

12. Continuous process for manufacturing a compressible, powdery saccharose composition such as defined in any one of claims 1 to 7, comprising the following steps:

- a drying step, by air, of a nebulised saccharose solution in an atomisation tower, the temperature of the air after drying and exiting said tower being comprised between 80 °C to 140 °C, for obtaining the glassy particles of saccharose,
- a stabilisation and crystallisation step of the glassy particles obtained from the drying step to obtain an essentially crystallised powdery composition at a temperature comprised between 50 °C to 80 °C and exhibiting a residual moisture content of less than or equal to 1% by weight based on the total weight of said composition, and
- a cooling step in air of the essentially crystallised powdery composition, to obtain the compressible, powdery saccharose composition, said composition being crystallised and being at a temperature of less than 30 °C, said stabilisation and crystallisation step being carried out by contacting said glassy particles with part of the recycled, compressible, powdery saccharose composition, the amount of recycle of said compressible, powdery saccharose composition varying from 30 to 70% by weight of dry matter of the nebulised saccharose solution.

13. Process according to any one of claims 8 to 12, wherein the drying step is carried out in the immediate vicinity of the nebulisation zone, especially in the upper part of the atomisation tower.

14. Process according to any one of claims 8 to 13, wherein the contacting step is carried out in the atomisation tower, under conditions such that the recycled composition is added at the level of a zone comprised between the first third and the second third of said atomisation tower, and especially at the level of the middle zone of said atomisation tower.

15. Process according to any one of claims 8 to 14, **characterised in that** the cooling step is carried out outside the atomisation tower.

16. Process according to any one of claims 8 to 15, **characterised in that** the cooling step is carried out under conditions enabling the residual moisture content of the essentially crystallised powdery composition to be reduced and also the crystallisation of said composition to be completed in order to obtain the compressible, powdery, crystallised saccharose composition.

17. Process according to any one of claims 8 to 16, wherein the initial saccharose solution has a dry matter content of 55 to 80% by weight.

18. Continuous process for manufacturing a compressible, powdery saccharose composition comprising at least 99.1% by weight of saccharose, exhibiting a residual moisture content of less than 0.1 % by weight, based on the total weight of said composition, said composition consisting of hollow particles, optionally agglomerated together in the form of granules, said process comprising:

- a drying step, by air, of a nebulised saccharose solution in an atomisation tower, the temperature of the air after drying and exiting said tower being comprised between 80 °C to 140 °C, for obtaining the glassy particles of saccharose,
- a stabilisation and crystallisation step of the glassy particles obtained from the drying step to obtain an essentially crystallised powdery composition at a temperature comprised between 50 °C to 80 °C and exhibiting a residual moisture content of less than or equal to 1% by weight based on the total weight of said composition, and
- an optional cooling step in air of the essentially crystallised powdery composition, to obtain the compressible, powdery saccharose composition, said composition being crystallised and being at a temperature of less than 30°C,

said stabilisation and crystallisation step being carried out by contacting said glassy particles with part of the recycled, essentially crystallised powdery composition or with part of the recycled, compressible, powdery saccharose composition, the amount of recycle of said essentially crystallised powdery composition or of the compressible, powdery saccharose composition ranging from 30 to 70% by weight of dry matter of the nebulised saccharose solution.

19. Continuous process for manufacturing a compressible, powdery saccharose composition comprising at least 99.1% by weight of saccharose, exhibiting a residual moisture content of less than 0.1 % by weight, based on the total weight of said composition, said composition consisting of hollow particles, optionally agglomerated together in the form of granules, said process comprising:

- a drying step, by air, of a nebulised saccharose solution in an atomisation tower, the temperature of the air after the drying and exiting said tower being comprised between 80 °C to 140 °C, for obtaining the glassy particles of saccharose, and
- a stabilisation and crystallisation step of the glassy particles obtained from the drying step to obtain an essentially crystallised, powdery composition at a temperature comprised between 50 °C to 80 °C and exhibiting a residual moisture content of less than or equal to 1% by weight based on the total weight of said composition,

said stabilisation and crystallisation step being carried out by contacting said glassy particles with part of the recycled, essentially crystallised powdery composition, the amount of recycle of said essentially crystallised powdery composition varying from 30% to 70% by weight of dry matter of the nebulised saccharose solution.

20. Continuous process for manufacturing a compressible, powdery saccharose composition comprising at least 99.1% by weight of saccharose, exhibiting a residual moisture content of less than 0.1 % by weight, based on the total weight of said composition, said composition consisting of hollow particles, optionally agglomerated together in the form of granules, said process comprising:

- a drying step, by air, of a nebulised saccharose solution in an atomisation tower, the temperature of the air after drying and exiting said tower being comprised between 80 °C to 140 °C, for obtaining the glassy particles of saccharose,
- a stabilisation and crystallisation step of the glassy particles obtained from the drying step to obtain an essentially crystallised powdery composition at a temperature comprised between 50 °C to 80 °C and exhibiting a residual moisture content of less than or equal to 1% by weight based on the total weight of said composition, and
- an cooling step in air of the essentially crystallised powdery composition, to obtain the compressible, powdery saccharose composition, said composition being crystallised and being at a temperature of less than 30 °C, said stabilisation and crystallisation step being carried out by contacting said glassy particles with part of the recycled, essentially crystallised powdery composition or with part of the recycled, compressible, powdery saccharose composition, the amount of recycle of said essentially crystallised powdery composition or of the compressible, powdery saccharose composition varying from 30 to 70% by weight of dry matter of the nebulised saccharose solution.

21. Continuous process for manufacturing a compressible, powdery saccharose composition comprising at least 99.1% by weight of saccharose, exhibiting a residual moisture content of less than 0.1% by weight, based on the total weight of said composition, said composition consisting of hollow particles, optionally agglomerated together in the form of granules, said process comprising:

  - a drying step, by air, of a nebulised saccharose solution in an atomisation tower, the temperature of the air after drying and exiting said tower being comprised between 80 °C to 140 °C, for obtaining the glassy particles of saccharose,
  - a stabilisation and crystallisation step of the glassy particles obtained from the drying step to obtain an essentially crystallised powdery composition at a temperature comprised between 50 °C to 80 °C and exhibiting a residual moisture content of less than or equal to 1% by weight based on the total weight of said composition, and
  - a cooling step in air of the essentially crystallised powdery composition to obtain the compressible, powdery saccharose composition, said composition being crystallised and being at a temperature of less than 30°C, said stabilisation and crystallisation step being carried out by contacting said glassy particles with part of the recycled, essentially crystallised powdery composition, the amount of recycle of said essentially crystallised powdery composition varying from 30 to 70% by weight of dry matter of the nebulised saccharose solution.

22. Continuous process for manufacturing a compressible, powdery saccharose composition comprising at least 99.1% by weight of saccharose, exhibiting a residual moisture content of less than 0.1 % by weight, based on the total weight of said composition, said composition consisting of hollow particles, optionally agglomerated together in the form of granules, said process comprising:

  - a drying step, by air, of a nebulised saccharose solution in an atomisation tower, the temperature of the air after drying and exiting said tower being comprised between 80 °C to 140 °C, for obtaining the glassy particles of saccharose,
  - a stabilisation and crystallisation step of the glassy particles obtained from the drying step to obtain an essentially crystallised powdery composition at a temperature comprised between 50 °C to 80 °C and exhibiting a residual moisture content of less than or equal to 1% by weight based on the total weight of said composition, and
  - a cooling step in air of the essentially crystallised powdery composition, to obtain the compressible, powdery saccharose composition, said composition being crystallised and being at a temperature of less than 30 °C, said stabilisation and crystallisation step being carried out by contacting said glassy particles with part of the recycled, compressible, powdery composition, the amount of recycle of said compressible, powdery saccharose composition varying from 30 to 70% by weight of dry matter of the nebulised saccharose solution.

23. Continuous process for manufacturing a compressible, powdery saccharose composition comprising 100% by weight of saccharose, exhibiting a residual moisture content of less than 0.1 % by weight, based on the total weight of said composition, said composition consisting of hollow particles, optionally agglomerated together in the form of granules, said process comprising:

  - a drying step, by air, of a nebulised saccharose solution in an atomisation tower, the temperature of the air used for drying and exiting said tower being comprised between 80 °C to 140 °C, for obtaining the glassy particles of saccharose,
  - a stabilisation and crystallisation step of the glassy particles obtained from the drying step, to obtain an essentially crystallised, powdery composition at a temperature comprised between 50 °C to 80 °C and exhibiting a residual moisture content of less than or equal to 1% by weight based on the total weight of said composition, and
  - a cooling step in air of the essentially crystallised powdery composition, to obtain the compressible, powdery saccharose composition, said composition being crystallised and being at a temperature of less than 30 °C, said stabilisation and crystallisation step being carried out by contacting said glassy particles with part of the recycled, essentially crystallised powdery composition or with part of the recycled, compressible, powdery saccharose composition, the amount of recycle of said essentially crystallised powdery composition or of the compressible, powdery saccharose composition varying from 30% to 70% by weight of dry matter of the nebulised saccharose solution.

**24.** Compressible, powdery sugar composition such as obtained by the process according to one of claims 18 to 23.

**25.** Use of a compressible, powdery sugar composition according to one of claims 1 to 7 or 24, for manufacturing compressed tablets, such as pharmaceuticals.

**26.** Use of a compressible, powdery sugar composition according to one of claims 1 to 7 or 24, for manufacturing powdery mixtures.

**27.** Use of a compressible, powdery sugar composition according to one of claims 1 to 7 or 24, for manufacturing fast-dissolving formulations.

**28.** Use of a compressible, powdery sugar composition according to one of claims 1 to 7 or 24, in a mixture with at least one other product, said other product being in soluble or powdery form, and being selected from among the following products: active principle, sweetener, colorant, aroma, enzyme or surfactant.

**29.** Use of a compressible, powdery sugar composition according to one of claims 1 to 7 or 24, for manufacturing sugar spheres, especially by coating and/or lamination and/or sugar coating.


**Patentansprüche**

**1.** Pulverförmige Zusammensetzung aus komprimierbarer Saccharose, die mindestens 99,1 Gew.-% Saccharose umfasst, die einen Restfeuchtigkeitsgehalt kleiner oder gleich 0,1 Gew.-%, bezogen auf das Gesamtgewicht der Zusammensetzung, aufweist, wobei die Zusammensetzung aus Hohlpartikeln besteht, die gegebenenfalls untereinander in Form von Körnchen agglomeriert sind.

**2.** Pulverförmige Zusammensetzung nach Anspruch 1, die mindestens 99,7 Gew.-% Saccharose umfasst.

**3.** Pulverförmige Zusammensetzung nach Anspruch 1 oder 2, **dadurch gekennzeichnet, dass** die durchschnittliche Korngrößenverteilung der Hohlpartikel, allein oder agglomeriert, zwischen 140 und 350 $\mu$m variiert.

**4.** Pulverförmige Zusammensetzung nach Anspruch 3, **dadurch gekennzeichnet, dass** der Variationskoeffizient der Partikel zwischen 35 und 55, insbesondere 40 und 50, variiert.

**5.** Pulverförmige Zusammensetzung nach irgendeinem der Ansprüche 1 bis 4, **dadurch gekennzeichnet, dass** sie eine scheinbare Dichte aufweist, die zwischen 0,45 und 0,75 variiert.

**6.** Pulverförmige Zusammensetzung nach irgendeinem der Ansprüche 1 bis 5, **dadurch gekennzeichnet, dass** sie eine Auslaufzeit von weniger als etwa 15 Sekunden aufweist.

**7.** Pulverförmige Zusammensetzung nach irgendeinem der Ansprüche 1 bis 6, **dadurch gekennzeichnet, dass** sie komprimiert werden kann,
wobei die Festigkeit des komprimierten Pulvers, das aus der pulverförmigen Zusammensetzung erhalten wurde, größer als etwa 1 MPa ist, wobei die Festigkeit für einen Pressdruck von mehr als etwa 15 kN gemessen wird, und das komprimierte Pulver einer Ausstoßkraft im Bereich zwischen etwa 50 bis etwa 80 N entspricht, wobei die Ausstoßkraft für einen Pressdruck im Bereich zwischen 15 und 20 kN gemessen wird.

**8.** Kontinuierliches Verfahren zur Herstellung einer pulverförmigen Zusammensetzung aus komprimierbarer Saccharose, wie in irgendeinem der Ansprüche 1 bis 7 definiert, umfassend:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten,
- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist, und

- gegebenenfalls eine Abkühlungsphase an Luft der im Wesentlichen kristallisierten pulverförmigen Zusammensetzung, um die pulverförmige Zusammensetzung aus komprimierbarer Saccharose zu erhalten, wobei die Zusammensetzung kristallisiert ist und eine Temperatur von weniger als 30 °C hat,

wobei die Stabilisierungs- und Kristallisationsphase durch Inkontaktbringen der Glaspartikel mit einem Teil der recycelten im Wesentlichen kristallisierten pulverförmigen Zusammensetzung oder mit einem Teil der recycelten pulverförmigen Zusammensetzung aus komprimierbarerer Saccharose durchgeführt wird, wobei die Recyclingrate der im Wesentlichen kristallisierten pulverförmigen Zusammensetzung oder der pulverförmigen Zusammensetzung aus komprimierbarer Saccharose zwischen 30 Gew.-% und 70 Gew.-% Trockenmasse der Lösung aus vernebelter Saccharose variiert.

9. Kontinuierliches Verfahren zur Herstellung einer pulverförmigen Zusammensetzung aus komprimierbarer Saccharose, wie in irgendeinem der Ansprüche 1 bis 7 definiert, umfassend:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten, und
- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist,

wobei die Stabilisierungs- und Kristallisationsphase durch das Inkontaktbringen der Glaspartikel mit einem Teil der recycelten im Wesentlichen kristallisierten pulverförmigen Zusammensetzung durchgeführt wird, wobei die Recyclingrate der im Wesentlichen kristallisierten pulverförmigen Zusammensetzung zwischen 30 Gew.-% und 70 Gew.-% Trockenmasse der Lösung aus vernebelter Saccharose variiert.

10. Kontinuierliches Verfahren zur Herstellung einer pulverförmigen Zusammensetzung aus komprimierbarer Saccharose, wie in irgendeinem der Ansprüche 1 bis 7 definiert, das die folgenden Schritte umfasst:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten,
- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist, und
- eine Abkühlungsphase an Luft der im Wesentlichen kristallisierten pulverförmigen Zusammensetzung, um die pulverförmige Zusammensetzung aus komprimierbarer Saccharose zu erhalten, wobei die Zusammensetzung kristallisiert ist und eine Temperatur von weniger als 30 °C hat,

wobei die Stabilisierungs- und Kristallisationsphase durch Inkontaktbringen der Glaspartikel mit einem Teil der recycelten im Wesentlichen kristallisierten pulverförmigen Zusammensetzung oder mit einem Teil der recycelten pulverförmigen Zusammensetzung aus komprimierbarerer Saccharose durchgeführt wird, wobei die Recyclingrate der im Wesentlichen kristallisierten pulverförmigen Zusammensetzung oder der pulverförmigen Zusammensetzung aus komprimierbarer Saccharose zwischen 30 Gew.-% und 70 Gew.-% Trockenmasse der Lösung aus vernebelter Saccharose variiert.

11. Kontinuierliches Verfahren zur Herstellung einer pulverförmigen Zusammensetzung aus komprimierbarer Saccharose, wie in irgendeinem der Ansprüche 1 bis 7 definiert, das die folgenden Schritte umfasst:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten,
- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist, und

- eine Abkühlungsphase an Luft der im Wesentlichen kristallisierten pulverförmigen Zusammensetzung, um die pulverförmige Zusammensetzung aus komprimierbarer Saccharose zu erhalten, wobei die Zusammensetzung kristallisiert ist und eine Temperatur von weniger als 30 °C hat,

wobei die Stabilisierungs- und Kristallisationsphase durch das Inkontaktbringen der Glaspartikel mit einem Teil der recycelten im Wesentlichen kristallisierten pulverförmigen Zusammensetzung'durchgeführt wird, wobei die Recyclingrate der im Wesentlichen kristallisierten pulverförmigen Zusammensetzung zwischen 30 Gew.-% und 70 Gew.-% Trockenmasse der Lösung aus vernebelter Saccharose variiert.

12. Kontinuierliches Verfahren zur Herstellung einer pulverförmigen Zusammensetzung aus komprimierbarer Saccharose, wie in irgendeinem der Ansprüche 1 bis 7 definiert, das die folgenden Schritte umfasst:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten,

- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist, und

- eine Abkühlungsphase an Luft der im Wesentlichen kristallisierten pulverförmigen Zusammensetzung, um die pulverförmige Zusammensetzung aus komprimierbarer Saccharose zu erhalten, wobei die Zusammensetzung kristallisiert ist und eine Temperatur von weniger als 30 °C hat,

wobei die Stabilisierungs- und Kristallisationsphase durch das Inkontaktbringen der Glaspartikel mit einem Teil der recycelten pulverförmigen Zusammensetzung aus komprimierbarer Saccharose durchgeführt wird, wobei die Recyclingrate der pulverförmigen Zusammensetzung aus komprimierbarer Saccharose zwischen 30 Gew.-% und 70 Gew.-% Trockenmasse der Lösung aus vernebelter Saccharose variiert.

13. Verfahren nach irgendeinem der Ansprüche 8 bis 12,
wobei die Trocknungsphase in unmittelbarer Nähe der Vernebelungszone, insbesondere im oberen Teil des Zerstäuberturms, durchgeführt wird.

14. Verfahren nach irgendeinem der Ansprüche 8 bis 13,
wobei die Phase des Inkontaktbringens im Zerstäuberturm unter Bedingungen durchgeführt wird, bei denen die recycelte Zusammensetzung in einer Zone im Bereich zwischen dem ersten Drittel und dem zweiten Drittel des Zerstäuberturms, und insbesondere in der mittleren Zone des Zerstäuberturms zugegeben wird.

15. Verfahren nach irgendeinem der Ansprüche 8 bis 14, **dadurch gekennzeichnet, dass** die Abkühlungsphase außerhalb des Zerstäuberturms durchgeführt wird.

16. Verfahren nach irgendeinem der Ansprüche 8 bis 15, **dadurch gekennzeichnet, dass** die Abkühlungsphase unter Bedingungen durchgeführt wird, die es ermöglichen, den Restfeuchtigkeitsgehalt der im Wesentlichen kristallisierten pulverförmigen Zusammensetzung zu senken und außerdem die Kristallisation der Zusammensetzung zu beenden, um die kristallisierte pulverförmige Zusammensetzung aus komprimierbarer Saccharose zu erhalten.

17. Verfahren nach irgendeinem der Ansprüche 8 bis 16,
wobei die Ausgangslösung aus Saccharose einen Trockenmasseanteil zwischen 55 Gew.-% und 80 Gew.-% aufweist.

18. Kontinuierliches Verfahren zur Herstellung einer pulverförmigen Zusammensetzung aus komprimierbarer Saccharose, die mindestens 99,1 Gew.-% Saccharose umfasst, die einen Restfeuchtigkeitsgehalt kleiner oder gleich 0,1 Gew.-%, bezogen auf das Gesamtgewicht der Zusammensetzung, aufweist, wobei die Zusammensetzung aus Hohlpartikeln besteht, die gegebenenfalls untereinander in Form von Körnchen agglomeriert sind, wobei das Verfahren umfasst:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten,

- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist, und

- gegebenenfalls eine Abkühlungsphase an Luft der im Wesentlichen kristallisierten pulverförmigen Zusammensetzung, um die pulverförmige Zusammensetzung aus komprimierbarer Saccharose zu erhalten, wobei die Zusammensetzung kristallisiert ist und eine Temperatur von weniger als 30 °C hat,

wobei die Stabilisierungs- und Kristallisationsphase durch Inkontaktbringen der Glaspartikel mit einem Teil der recycelten im Wesentlichen kristallisierten pulverförmigen Zusammensetzung oder mit einem Teil der recycelten pulverförmigen Zusammensetzung aus komprimierbarerer Saccharose durchgeführt wird, wobei der Recyclingrate der im Wesentlichen kristallisierten pulverförmigen Zusammensetzung oder der pulverförmigen Zusammensetzung aus komprimierbarer Saccharose zwischen 30 Gew.-% und 70 Gew.-% Trockenmasse der Lösung aus vernebelter Saccharose variiert.

19. Kontinuierliches Verfahren zur Herstellung einer pulverförmigen Zusammensetzung aus komprimierbarer Saccharose, die mindestens 99,1 Gew.-% Saccharose umfasst, die einen Restfeuchtigkeitsgehalt kleiner oder gleich 0,1 Gew.-%, bezogen auf das Gesamtgewicht der Zusammensetzung, aufweist, wobei die Zusammensetzung aus Hohlpartikeln besteht, die gegebenenfalls untereinander in Form von Körnchen agglomeriert sind, wobei das Verfahren umfasst:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten, und

- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist,

wobei die Stabilisierungs- und Kristallisationsphase durch das Inkontaktbringen der Glaspartikel mit einem Teil der recycelten im Wesentlichen kristallisierten pulverförmigen Zusammensetzung durchgeführt wird, wobei die Recyclingrate der im Wesentlichen kristallisierten pulverförmigen Zusammensetzung zwischen 30 Gew.-% und 70 Gew.-% Trockenmasse der Lösung aus vernebelter Saccharose variiert.

20. Kontinuierliches Verfahren zur Herstellung einer pulverförmigen Zusammensetzung aus komprimierbarer Saccharose, die mindestens 99,1 Gew.-% Saccharose umfasst, die einen Restfeuchtigkeitsgehalt kleiner oder gleich 0,1 Gew.-%, bezogen auf das Gesamtgewicht der Zusammensetzung, aufweist, wobei die Zusammensetzung aus Hohlpartikeln besteht, die gegebenenfalls untereinander in Form von Körnchen agglomeriert sind, wobei das Verfahren umfasst:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten,

- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist, und

- eine Abkühlungsphase an Luft der im Wesentlichen kristallisierten pulverförmigen Zusammensetzung, um die pulverförmige Zusammensetzung aus komprimierbarer Saccharose zu erhalten, wobei die Zusammensetzung kristallisiert ist und eine Temperatur von weniger als 30 °C hat,

wobei die Stabilisierungs- und Kristallisationsphase durch Inkontaktbringen der Glaspartikel mit einem Teil der recycelten im Wesentlichen kristallisierten pulverförmigen Zusammensetzung oder mit einem Teil der recycelten pulverförmigen Zusammensetzung aus komprimierbarerer Saccharose durchgeführt wird, wobei die Recyclingrate der im Wesentlichen kristallisierten pulverförmigen Zusammensetzung oder der pulverförmigen Zusammensetzung aus komprimierbarer Saccharose zwischen 30 Gew.-% und 70 Gew.-% Trockenmasse der Lösung aus vernebelter Saccharose variiert.

21. Kontinuierliches Verfahren zur Herstellung einer pulverförmigen Zusammensetzung aus komprimierbarer Saccharose, die mindestens 99,1 Gew.-% Saccharose umfasst, die einen Restfeuchtigkeitsgehalt kleiner oder gleich 0,1 Gew.-%, bezogen auf das Gesamtgewicht der Zusammensetzung, aufweist, wobei die Zusammensetzung aus Hohlpartikeln besteht, die gegebenenfalls untereinander in Form von Körnchen agglomeriert sind, wobei das Verfahren umfasst:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten,
- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist, und
- eine Abkühlungsphase an Luft der im Wesentlichen kristallisierten pulverförmigen Zusammensetzung, um die pulverförmige Zusammensetzung aus komprimierbarer Saccharose zu erhalten, wobei die Zusammensetzung kristallisiert ist und eine Temperatur von weniger als 30 °C hat,

wobei die Stabilisierungs- und Kristallisationsphase durch das Inkontaktbringen der Glaspartikel mit einem Teil der recycelten im Wesentlichen kristallisierten pulverförmigen Zusammensetzung durchgeführt wird, wobei die Recyclingrate der im Wesentlichen kristallisierten pulverförmigen Zusammensetzung zwischen 30 Gew.-% und 70 Gew.-% Trockenmasse der Lösung aus vernebelter Saccharose variiert.

22. Kontinuierliches Verfahren zur Herstellung einer pulverförmigen Zusammensetzung aus komprimierbarer Saccharose, die mindestens 99,1 Gew.-% Saccharose umfasst, die einen Restfeuchtigkeitsgehalt kleiner oder gleich 0,1 Gew.-%, bezogen auf das Gesamtgewicht der Zusammensetzung, aufweist, wobei die Zusammensetzung aus Hohlpartikeln besteht, die gegebenenfalls untereinander in Form von Körnchen agglomeriert sind, wobei das Verfahren umfasst:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten,
- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist, und
- eine Abkühlungsphase an Luft der im Wesentlichen kristallisierten pulverförmigen Zusammensetzung, um die pulverförmige Zusammensetzung aus komprimierbarer Saccharose zu erhalten, wobei die Zusammensetzung kristallisiert ist und eine Temperatur von weniger als 30 °C hat,

wobei die Stabilisierungs- und Kristallisationsphase durch das Inkontaktbringen der Glaspartikel mit einem Teil der recycelten pulverförmigen Zusammensetzung aus komprimierbarer Saccharose durchgeführt wird, wobei die Recyclingrate der pulverförmigen Zusammensetzung aus komprimierbarer Saccharose zwischen 30 Gew.-% und 70 Gew.-% Trockenmasse der Lösung aus vernebelter Saccharose variiert.

23. Kontinuierliches Verfahren zur Herstellung einer pulverförmigen Zusammensetzung aus komprimierbarer Saccharose, die 100 Gew.-% Saccharose umfasst, die einen Restfeuchtigkeitsgehalt kleiner oder gleich 0,1 Gew.-%, bezogen auf das Gesamtgewicht der Zusammensetzung, aufweist, wobei die Zusammensetzung aus Hohlpartikeln besteht, die gegebenenfalls untereinander in Form von Körnchen agglomeriert sind, wobei das Verfahren umfasst:

- eine Trocknungsphase mittels Luft einer Lösung aus vernebelter Saccharose in einem Zerstäuberturm, wobei die Temperatur der Luft, die der Trocknung diente und aus dem Turm austritt, im Bereich zwischen 80 °C und 140 °C liegt, um Glaspartikel aus Saccharose zu erhalten,
- eine Stabilisierungs- und Kristallisationsphase der Glaspartikel, die am Ende der Trocknungsphase erhalten wurden, um eine im Wesentlichen kristallisierte pulverförmige Zusammensetzung bei einer Temperatur im Bereich zwischen 50 °C und 80 °C zu erhalten, und die einen Restfeuchtigkeitsgehalt von weniger oder gleich 1 Gew.-%, bezogen auf das Gesamtgewicht der Zusammensetzung, aufweist, und

- eine Abkühlungsphase an Luft der im Wesentlichen kristallisierten pulverförmigen Zusammensetzung, um die pulverförmige Zusammensetzung aus komprimierbarer Saccharose zu erhalten, wobei die Zusammensetzung kristallisiert ist und eine Temperatur von weniger als 30 °C hat,

wobei die Stabilisierungs- und Kristallisationsphase durch Inkontaktbringen der Glaspartikel mit einem Teil der recycelten im Wesentlichen kristallisierten pulverförmigen Zusammensetzung oder mit einem Teil der recycelten pulverförmigen Zusammensetzung aus komprimierbarerer Saccharose durchgeführt wird, wobei die Recyclingrate der im Wesentlichen kristallisierten pulverförmigen Zusammensetzung oder der pulverförmigen Zusammensetzung aus komprimierbarer Saccharose zwischen 30 Gew.-% und 70 Gew.-% Trockenmasse der Lösung aus vernebelter Saccharose variiert.

24. Pulverförmige Zusammensetzung aus komprimierbarem Zucker, wie durch das Verfahren nach einem der Ansprüche 18 bis 23 erhalten.

25. Verwendung einer pulverförmigen Zusammensetzung aus komprimierbarem Zucker nach einem der Ansprüche 1 bis 7 oder 24 zur Herstellung von Tabletten, insbesondere Pharmazeutika.

26. Verwendung einer pulverförmigen Zusammensetzung aus komprimierbarem Zucker nach einem der Ansprüche 1 bis 7 oder 24 zur Herstellung von pulverförmigen Gemischen.

27. Verwendung einer pulverförmigen Zusammensetzung aus komprimierbarem Zucker nach einem der Ansprüche 1 bis 7 oder 24 zur Herstellung von schnelllöslichen Formulierungen.

28. Verwendung einer pulverförmigen Zusammensetzung aus komprimierbarem Zucker nach einem der Ansprüche 1 bis 7 oder 24 als Gemisch mit mindestens einem anderen Produkt, wobei das andere Produkt in löslicher oder pulverförmiger Form vorliegen kann und aus den folgenden Produkten ausgewählt ist: Wirkstoff, Süßstoff, Farbstoff, Aroma, Enzym oder grenzflächenaktives Mittel.

29. Verwendung einer pulverförmigen Zusammensetzung aus komprimierbarem Zucker nach einem der Ansprüche 1 bis 7 oder 24 zur Herstellung von Zuckerkugeln, insbesondere durch Coating und/oder Laminieren und/oder Dragieren.

FIGURE 1

**Comparaison Sucres atomisés**

FIGURE 2

EP 1 941 062 B1

**FIGURE 3**

EP 1 941 062 B1

FIGURE 4A

FIGURE 4B

## RÉFÉRENCES CITÉES DANS LA DESCRIPTION

**Documents brevets cités dans la description**

- EP 0334617 A
- WO 0206538 A
- GB 1240691 A
- GB 1282878 A
- GB 1386378 A
- GB 1386379 A
- GB 1387062 A
- WO 03000936 A
- WO 03000396 A